(19) Europäisches Patentamt
European Patent Office
Office européen des brevets

(11) **EP 4 141 095 A1**

(12) **EUROPEAN PATENT APPLICATION**

(43) Date of publication:
**01.03.2023 Bulletin 2023/09**

(21) Application number: **21306143.5**

(22) Date of filing: **25.08.2021**

(51) International Patent Classification (IPC):
**C12M 1/12** (2006.01)          **C12M 1/26** (2006.01)
**B33Y 70/10** (2020.01)

(52) Cooperative Patent Classification (CPC):
**C12M 25/14; B29C 64/106; B33Y 10/00;
B33Y 70/00; B33Y 80/00; C12M 33/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Sartorius Stedim FMT SAS
13400 Aubagne (FR)**
• **UNIVERSITE CLAUDE BERNARD - LYON 1
69100 Villeurbanne (FR)**
• **Institut National des Sciences Appliquées de
Lyon
69621 Villeurbanne Cedex (FR)**
• **Ecole Superieure de Chimie Physique
Electronique de Lyon
69616 Villeurbanne, Cedex (FR)**

• **Le Centre National de la Recherche Scientifique
75794 Paris Cedex 16 (FR)**

(72) Inventors:
• **CHASTAGNIER, Laura
69100 Villeurbanne (FR)**
• **PETIOT, Emma
69100 Villeurbanne (FR)**
• **COURTIAL, Edwin Geoffrey
69100 Villeurbanne (FR)**
• **MARQUETTE, Christophe
69100 Villeurbanne (FR)**

(74) Representative: **Mewburn Ellis LLP
Aurora Building
Counterslip
Bristol BS1 6BX (GB)**

(54) **PRODUCTION OF CELLULAR PRODUCTS BY CELLS EMBEDDED IN A HYDROGEL MATRIX**

(57) Methods and systems for performing a bioproduction process are described, the bioproduction process comprising the production of a cellular product by a cell population. The methods include the steps of culturing the cell population at least partially embedded in a hydrogel matrix forming a three-dimensional structure.

Fig. 1A

**Description**

Field of the Present Disclosure

The present disclosure relates to methods for the industrial production of cellular products such as cells, parts or products of cells, wherein the cells are included in a 3D printed hydrogel matrix. Related methods, systems and products are described.

Background

Bioprocesses use biological systems to produce a specific biomaterial, for example a biomolecule with therapeutic effects. This process typically involves placing cells and/or microbes into a bioreactor with media containing nutrients under controlled atmospheric conditions. In the context of bioproduction, bioprocesses are typically performed as large-scale culture processes in a stirred tank bioreactor comprising liquid medium in which the cells are in suspension or cultured on microcarriers. More recently, adherent cell culture processes for bioproduction have been proposed in which the cells grow adhering to the surface of a scaffold, such as compacted fabric pieces or microporous carriers. These production types may be referred to as "fixed-bed bioreactor". Both the stirred tank bioreactor processes and the fixed-bed bioreactor processes impose some physical limitations on the cellular density that can be achieved, and hence ultimately the productivity of the system. Indeed, in stirred cultures (whether with cells in suspension or on microcarriers), the supply of gasses and nutrients to the cells becomes limiting as the cell density increases, as the supply and agitation means can damage the cells if operated at high rates (e.g. due to the shear stress caused by the moving medium and/or rotating shaft). Thus, there is a trade-off between the density of cells that can be effectively sustained and the physical damage caused by the systems that provide the gasses and nutrients to sustain such densities. Considering that production cells typically have high oxygen and nutrient requirements, partially because they have been selected for high growth and production rates, this can quickly become problematic. This problem is often mitigated by introducing compounds that protect the cells from hydrodynamic shear stress (such as e.g. polymeric surfactants), which may cause problematic in downstream processing. Additionally, the recovery of biomaterials from such cultures require filtering of the cells from the culture medium (a process called "clarification"), which can negatively impact both the viability of the cells (which is especially problematic if it needs to be performed inline as this can reduce the productivity of the culture) and the quality of the product.

In fixed-bed bioreactors, the cell density is typically limited by the presence of the scaffold itself, which occupies much of the space in the reactor. Additionally, seeding of the cells onto the scaffold is typically performed by flow distribution, resulting in a heterogeneous distribution of the cells on the scaffold. This does not result in optimal colonisation of the space, thus reducing productivity. Further, cell recovery from carriers is a complex process that often damages the cells. This is particularly problematic when the cells themselves are a valuable product. Further, culture on carriers typically makes use of complex culture medium additives such as serum , in order to increase cell adhesion. This increases the complexity of the process for recovery of the product, particularly in the case of secreted proteins, and often negatively impacts the purity of the product.

Therefore, a need exists for improved systems and methods for the production of cellular products, which do not suffer from the all of the drawbacks of the prior art.

Summary

The present inventors recognised that the production of cellular products in a 3D printed hydrogel matrix could solve many of the problems faced by traditional bioproduction methods in stirred bioreactors or fixed-bed bioreactors, achieving very high cell densities with production cells, and enabling efficient recovery of any types of cellular products including the cells themselves or a secreted product thereof. The present inventors have previously developed a bioink formulation for 3D printing of tissues, in particular skin substitutes (US 2019/0002836; Pourchet et al., Adv. Healthcare Mater. 2017, 1601101). For this purpose a solution comprising gelatin, alginate and fibrinogen together with fibroblasts was 3D printed into a thin solid structure and then keratinocytes were seeded onto the structure. This produced a skin substitute, with a strong accumulation of the fibroblasts at the interface between the printed object and the culture medium, forming a good substrate for the seeding of keratinocytes. These structures, including the support and the cell layers grown onto it, can be implanted directly as a skin substitute. More complex, porous structures such as a latticed cube were also evaluated for the production of larger amounts of skin substitutes without vascularisation, through improved culture medium perfusion (Pourchet et al., Bioprinting, Volume 20, December 2020, Pages e00114). The inventors hypothesised that such a system could be used in a bioproduction process (i.e. to produce cellular products) rather than for tissue engineering. This a substantially different context as production cells have very different requirements from primary cells and fibroblasts used in tissue engineering, and the requirements of a bioprocess (production of a cellular product in maximal amounts while maintaining specific quality attributes) are substantially different from the requirements of a tissue substitute. In particular, production cells typically have higher growth and metabolic rates and grow as spheroids rather than as monolayers on the surface of structures like fibroblasts. Further, in the context of a bioprocess maximising cell density and/or productivity is an important goal, whereas replicating the structure of a native tissue is the main goal in tissue engineering. Thus, a system that has been designed to grow a tissue may not be suitable (let alone advantageous) in the context of a bioproduction process. The inventors designed a process whereby cells that are expected to grow as single cells / spheroids rather than continuous tissues were formulated in a printable bioink (hydrogel), printed as a 3D structure, thereby forming a structure in which the cells are embedded ("cellularised structure"), and cultured for the production of a cellular product. The inventors found that not only was such as culture in 3D bioprinted hydrogel suitable for such cells, it additionally presented many advantages. For example, the culture can enable very high cell densities to be reached at least in part due to homogeneous seeding, extensive colonisation of the cellularised structure, and growth in the protective environment of the cellularised object thereby enabling the supply of nutrients at levels sufficient to sustain high cell densities. Additionally, the recovery of the cellular product was greatly improved at least in part due to efficient recovery of products secreted in the culture medium (with limited cellular damage even inline) and efficient recovery of cells embedded in the structure with limited cell viability loss.

According to a first aspect of the disclosure, there is provided a method for performing a bioproduction process comprising the production of a cellular product by a cell population, the method including the step of culturing the cell population at least partially embedded in a hydrogel matrix forming a three-dimensional structure. A three-dimensional structure refers to an object that has a 3D shape that is not merely a solid object having exclusively smooth surfaces (such as e.g. an object having exclusively flat surfaces and a certain constant thickness such as a parallelepiped). For example, a three-dimensional structure may be an object that has an external shape and an internal structure (such as e.g. one or more pores or cavities), and/or an external shape comprising one or more protrusions on at least one of its surfaces. In the context of the invention, such structures are typically 3D printed. This has many advantages as will be described further below, as it enables greater flexibility of design of the structure and enables the creation of pores / cavities of controlled shapes within the structure. According to the present invention, the hydrogel matrix is not provided on a 3D structure, it is the hydrogel matrix itself that forms the 3D structure.

The method of the first aspect may have any one or any combination of the following optional features.

The hydrogel matrix may have been obtained by providing a bioink composition comprising one or more biomaterials capable of forming a hydrogel and a cell population in a controlled three-dimensional shape to obtain a cellularised structure. The provision of the cell population as part of a bioink comprising the biomaterial(s) that form the hydrogel matrix may advantageously enable a tight control of the cell seeding such that this process is homogeneous throughout the structure. By contrast, in fixed bed bioreactors cell seeding is typically performed by flowing a solution of cells over the support, which typically results in a heterogenous cell seeding. This in turn may impact the cell density that is achievable in the process through local density limitation effects. By contrast, according to the present invention the cell seeding process may be much more homogeneous, thereby making it possible for the cell population to colonise the hydrogel structure with limited local density restrictions (until maximal cell density has been reached throughout the structure). Further, the provision of the cell population as part of a bioink comprising the biomaterial(s) that form the hydrogel matrix may advantageously enable the cells to be embedded in the hydrogel matrix at least at the start of the culture process, providing a protective structure for the cells to grow and colonise. The bioink composition may comprise a liquid culture medium. The bioink composition may have been obtained by mixing one or more solutions each comprising biomaterials capable of forming a hydrogel and/or cells in a liquid culture medium. The use of culture medium in the bioink composition advantageously results in improved growth rates and ultimately improved cell densities in the cellularised structure. The culture medium use may comprise conditioned medium (i.e. culture medium in which the cells were cultured prior to formulation of the bioink).

The cell population may comprise or consist of cells from one or more cell lines. The cell population may not comprise primary cells. The cell population may comprise or consist of cells from a single cell line. The cell population may grow as single cells or clusters of cells such as spheroids. The cell population may not form a tissue in or on the hydrogel structure. The cell population may comprise or consist of cells from one or more production cell lines. The one or more production cell lines may be selected from: an MDCK cell line, AGE.CR1™, PRE.C6, a VERO cell line, EB.14, EP.66™, HEK293, BHK21, a CHO cell line, NS0, Sp2, Sf9, SF21, MRC-5, WI-38, a CEF cell line, and a hybridoma cell line. The one or more production cell lines are selected from a VERO cell line, a CHO cell line, an MDCK cell line, and HEK293. Production cells typically have been optimised for growth and/or productivity (e.g. protein expression rates) and hence have different characteristics and culture requirements from other cells such as primary cells. For example, production cells may have higher metabolic requirements (e.g. they may require more nutrients and/or oxygen in order to survive). Production cells typically grow as single cells or spheroids, whereas at least some primary cells and other cells have the ability to form organised structures such as organoids or tissues. Indeed, many production cell lines have been selected to be compatible with culture in suspension, and/or to grow as homogeneous population without directional/functional specialisation. Thus, the requirements of production cells in terms of physical support, supply of nutrients/gasses, etc. may be very different from that of other cells. The present inventors have discovered that production cells are particularly suitable for use in the bioproduction process according to the invention, as their high growth rates and/or production rates can be optimally exploited in this context.

The three-dimensional structure may have one or more internal cavities. The three-dimensional structure may form a porous structure. The one or more internal cavities or pores may have a controlled size distribution. The one or more internal cavities or pores may have a size within a predetermined range of sizes. The three-dimensional structure may comprise a plurality of internal cavities arranged on a regular lattice. As the cell population grows, the cells may at least partially or fully colonise the internal cavities / pores within the three-dimensional structure. Thus, the method may comprise maintaining the cell population until it reaches a desired density, wherein at the desired density the cells at least partially occupy the internal cavities / pores of the three dimensional structure. The one or more internal cavities or pores may have dimensions between 0.1 and 2 $mm^2$, where these dimensions refer to the surface area of the cross section of pores forming a channel through the bioprinted object. The present inventors have found such dimensions to be particularly suitable for the high density culture of production cells. The three dimensional structure may not contain internal cavities. This is particularly suitable for three dimensional structures that are maintained in suspension in a liquid culture medium. Such three dimensional structures are typically smaller than three dimensional structures that are maintained on a support in a vessel. Embodiments comprising a three dimensional structure maintained on a support may advantageously be porous to enable an improved flow of culture medium throughout the structure, thereby ensuring that cells throughout the structure are adequately supplied with nutrients and gases.

The three-dimensional structure may have been obtained by additive manufacturing. The production of the three-dimensional structure by additive manufacturing is particularly advantageous as it enables the precise control of the porosity of the three-dimensional structure, as well as the easy provision of a structure that has a different shape (whether overall or internal shape, such as e.g. porosity, total volume, etc.) for any use case, such as e.g. depending on the nature of the cell population, the composition of the hydrogel, the configuration of the bioreactor in which the structure is maintained (e.g. size, production scale, etc.). By contrast, the porosity in packed reactors (fixed bed reactors) is typically poorly controlled, resulting in a lack of homogeneity of the cell culture conditions and possibly an under-utilisation of the volume of culture as some pores will be too small or too large, and homogeneous provision of nutrients and gasses throughout the packed bed is hindered by the heterogeneity in pore size. In the present system, homogeneous pores can be produced, which can be arranged in a regular or at least homogeneous pattern, thereby increasing the homogeneity of the flow of liquid (carrying nutrients and gasses) through the cellularised object. The method may comprise obtaining a three-dimensional structure comprising a hydrogel matrix by additive manufacturing. Obtaining a three-dimensional structure by additive manufacturing may comprise depositing a composition at a rate below 0.2 $mm^3$/s. Obtaining a three-dimensional structure by additive manufacturing may comprise depositing a composition as a filament. The filament may have a diameter between 200 and 800 $\mu$m. The three-dimensional structure may comprise a plurality of cavities arranged on a regular lattice. The regular lattice may be a cube lattice. The use of a lattice may advantageously enable to produce a homogeneous and controllable flow of culture medium through the structure. The three-dimensional structure may have dimensions such that the total (external) volume of the three-dimensional structure is in the order of $cm^3$ (e.g. 1 to 10 $cm^3$) or $dm^3$ (e.g. 1 to 10 $dm^3$). For example, the three-dimensional structure may have been obtained by depositing between 1 ml and 1l of bioink, between 1 ml and 500 ml of bioink or between 1 ml and 250 ml of bioink. Such three dimensional structures may be supported on a surface. Alternatively, the three-dimensional structure may have dimensions such that the total (external) volume of the three-dimensional structure is in the order of $\mu$m$^3$ (e.g. structures with an external diameter of between 50 $\mu$m and 500 $\mu$m, or with an external volume of 65.10$^3$ to 65.10$^6$ $\mu$m$^3$ or 0.065 $mm^3$) to $mm^3$ (e.g. 1 to 1000 $mm^3$). Such three dimensional structures may be cultured in suspension. Such three dimensional structures may be deposited as droplets rather than filaments. The three-dimensional structure may be deposited as a filament, such as e.g. by extrusion (e.g. pneumatic extrusion). The filament may have a substantially constant diameter between 200 and 800 $\mu$m. The present inventors have found such dimensions to be particularly advantageous in order to support the maintenance of cells at least partially embedded within the hydrogel matrix forming the filament. In particular, such dimensions resulted in particularly high cellular densities as they struck a good balance between structural integrity to support the growth of cells, providing sufficient surface area for the cells to grow on, and limiting the volume of matrix in which the cells can grow but do so typically to a lower extent. In other words, the inventors have observed that production cells can grow at least partially embedded within the hydrogel matrix, with a larger proportion of the grow occurring supported by the matrix but external to it than within the matrix body. The inventors have further observed that the above-mentioned filament dimensions advantageously enabled the cells to optimally grow both within and on the matrix. The composition may have been deposited at a printing rate below 0.2 $mm^3$/s. This may advantageously result in a low shear rate during printing, thereby increasing cell viability.

The method may comprises providing a bioink composition, depositing the bioink composition in a controlled three-dimensional shape to obtain a cellularised structure and/or consolidating the deposited bioink composition. Consolidating the bioink composition may comprise exposing the deposited bioink composition to one or more solutions that cross-link one or more of the biomaterials capable of forming a hydrogel. Providing the bioink composition may comprise incubating the bioink composition at a predetermined temperature for a predetermined period of time such that the rheological properties of the bioink composition are compatible with printing without loss of cell viability. The step of providing the bioink composition may comprise a step of mixing a solution comprising cells in suspension and one or more solutions comprising biomaterials capable of forming a hydrogel. The step of providing the bioink composition may comprise one or more steps selected from: preparing one or more solutions comprising biomaterials capable of forming a hydrogel, amplifying a seed cell population, harvesting an amplified cell population, obtaining a solution comprising cells in suspension (such as e.g. by re-suspending harvested cells). Consolidating the bioink composition may comprise exposing the deposited bioink composition to one or more solutions that cross-link one or more of the biomaterials capable of forming a hydrogel. For example, when the biomaterial capable of forming a hydrogel is alginate, consolidating the bioink composition may comprise exposing the deposited bioink composition to a solution that comprises calcium ions. As another example, when the biomaterial capable of forming a hydrogel is fibrinogen, consolidating the bioink composition may comprise exposing the deposited bioink composition to a solution that comprises thrombin. Providing the bioink composition may comprise incubating the bioink composition at a predetermined temperature for a predetermined period of time such that the rheological properties of the bioink composition are compatible with printing without loss of cell viability. Rheological properties that are compatible with printing and cell viability may be defined by defining a maximum static yield stress and/or maximum shear stress that does not result in substantial loss of viability, and a minimum static yield stress and/or maximum shear stress that results in a composition that can be deposited with a required level of shape fidelity. These may enable the skilled person to determine, for a composition having a particular set of rheological properties (such as e.g. as determined by obtaining a rheogram for the composition and comparing it to a reference rheogram), a range of temperatures that are compatible with printing and cell viability. For example, the bioink composition may be incubated for a set period of time at a temperature is below a temperature that would result in a loss of cell viability and that results in rheological properties that are compatible with printing and cell viability, such as e.g. a temperature at which the viscosity of the bioink composition is increased bioink compared to a temperature at which the bioink composition was prepared. For example, the bioink composition may be incubated at a temperature between 21 and 28C, such as e.g. approximately 21 C, in order to increase the viscosity of the composition to enable the bioink composition to be printed into an object that maintains its shape, after preparation of the bioink composition at approximately 37C (at which temperature the components of the bioink composition may be soluble and the composition may be fluid). It is advantageous for the viscosity of the bioink composition to be within a range that enables printing (i.e. such that a filament can be printed and maintain its shape) while not being so low as to increasing the shear rate experienced by the cells during printing to a level that would result in a loss of viability. The exemplary temperatures and compositions described herein are believed to have this effect, although alternative combinations of concentrations of components and temperature may be identified that would have the same effect. For example, increasing the concentration of gelatin may result in a bioink composition that would be incubated at a higher temperature to have the same viscosity. Further, the requirement for the bioink composition to maintain its shape when deposited may vary depending on the shape that is deposited (e.g. porous shapes may require higher shape fidelity than non-porous / solid objects) and the deposition configuration (e.g. supported shapes such as e.g. moulded shapes may not require high shape fidelity). The alginate may be low viscosity alginate. This may ensure that the viscosity of the bioink composition is not too high during printing. The method may further comprise determining the rheological properties of the bioink composition prior to deposition, for example by obtaining a rheogram of the bioink composition. The rheogram may then be compared with a reference rheogram to ensure that the bioink composition has desired rheological properties.

The hydrogel matrix may comprise alginate and fibrin. The hydrogel matrix may have been obtained by consolidation of a composition comprising alginate, fibrinogen and gelatin. The composition may comprise between 1.75% and 26% w/v of gelatin, between 0.15% and 4.2% w/v of alginate, and between 0.15% and 5.25% w/v of fibrinogen. Consolidation of the composition may comprise exposing the composition to a calcium salt and thrombin, and/or exposing the composition to transglutaminase. The composition may be deposited in conditions enabling the gelatin in the composition to solidify thereby at least temporarily maintaining the 3D structure of the deposited composition. Hydrogel matrices comprising alginate and fibrin were found by the inventors to have a variety of properties that make them particularly suitable for the long term culture of production cells. For example, they are non-toxic to the cells, stable in culture medium for even long periods of time (both structurally and chemically), enable production cells to colonise the hydrogel structure (i.e. they do not hinder growth of such cells and do not require additional supplements in the culture medium to enable the cells to colonise the support, even for cells that are typically grown in suspension, in particular, the presence of fibrin promotes cell adhesion), and can be consolidated in conditions compatible with cell viability. Further, solutions comprising gelatin have the additional benefit that they can be 3D printed (or otherwise shaped) in conditions compatible with cell viability, prior to consolidation, for example by depositing or otherwise forming the object in conditions below the melting temperature of the gelatin (e.g. depositing the composition onto a cooled plate). The gelatin component can additionally be removed after consolidation, for example by incubating the object at a temperature above the melting temperature of the gelatin. Suitable compositions, preparations and printing processes are described in US 2019/002836 A1. However, according to the present invention (and contrary to what is disclosed in US 2019/002836 A1), it is preferable for the bioink compositions used herein to be prepared using culture medium as the buffer/solution used to prepare at least some of the components (preferably all of the components) of the bioink composition. Advantageously, such compositions are further compatible with observation of the cell population without separating the cells from the hydrogel matrix, such as e.g. during the course of the bioprocess. This may advantageously enable the monitoring of the culture, for example using optical and/or fluorescence microscopy. Thus, the method may comprise monitoring the bioprocess during the cell culture by examining the cell population at least partially embedded in the hydrogel matrix using optical and/or fluorescence microscopy.

The cell population and hydrogel matrix structure may together form a cellularised structure and culturing the cell population at least partially embedded in a hydrogel matrix forming a three-dimensional structure may comprise maintaining the cellularised structure in a liquid culture medium in a bioreactor. The cellularised structure may be completely immersed in the culture medium. The culture medium may be a chemically defined culture medium. The culture medium may be a serum free culture medium. The culture medium may be a protein-free culture medium. The culture medium may be free of additives used to protect the cells from hydrodynamic damage. The culture medium may be free of detergents. The culture medium may be free of calcium. In particular, if the bioink composition contains alginate, the culture media may be calcium free to prevent alginate chelation with calcium ions. Conventional cell based production process often necessitate the use of additives to protect the cells from hydrodynamic stress. For example, additives such as pluronic (a Polyoxyethylene-polyoxypropylene block copolymer, a non-ionic detergent that is commonly used to protect cells from hydrodynamic damage) are frequently used in conventional stirred bioreactor bioprocesses. In the context of the present invention, such additives may not be necessary as the cells are protected by the hydrogel matrix and a flow can be used to provide the cells with the necessary gasses and nutrients instead of a rotary shaft and propellor as in common stirred bioreactor cultures. Many conventional cell based production process require the addition of serum or other complex protein-containing mixtures, potentially of undefined chemical composition, in order to ensure the growth/fitness of the cells in the stress conditions of a stirred bioreactor, or to promote adhesion to a surface or carrier. In the context of the present invention, such additives may not be necessary are the cells are protected by the hydrogel matrix in which they are at least partially embedded. The method may comprise maintaining the cell population until it reaches a predetermined cell density. The predetermined cell density may be at least $10^8$ cell/ml, at least $10^9$ or at least $10^{10}$ cell/ml. The cellular density may refer to the number of cells per unit of volume of the composition used to form the three-dimensional structure. The volume of the three-dimensional structure may be equivalent to the volume of the composition used to form the three-dimensional structure in the case of a non-porous structure. In the case of a porous structure, the volume of the three-dimensional structure may be higher than the volume of the composition used to form the three-dimensional structure. For example, a three-dimensional structure that has a 50% porosity may comprise a total volume of composition that is half of the total volume of the three-dimensional structure. Thus, the number of cells per unit of volume of the three-dimensional structure may be half the cellular density expressed as a number of cells per unit of volume of composition used to form the three-dimensional structure. Further, the number of cells obtained may also be expressed relative to the volume of culture medium in the culture vessel, based on the cell density and the ratio between the volume of the composition used to form the three-dimensional structure (or the volume of the three dimensional structure and its porosity) and the volume of medium in the culture vessel. For example, using a known ratio between the volume of medium in the culture vessel and the volume of the composition used to form the three-dimensional structure (e.g. between 5 and 50), the number of cells may be expressed per unit of volume of the culture medium by dividing the cellular density by said ratio. Cellular densities of at least $10^8$ cell/ml, at least $10^9$ or at least $10^{10}$ cell/ml may therefore be equivalent to at least 0.2 to $0.002 \times 10^8$ cell/ml, at least 0.2 to $0.002 \times 10^9$ or at least 0.2 to $0.002 \times 10^{10}$ cell/ml of culture medium. The cellular density may be expressed as a number of cell per unit of volume (such as e.g. ml, $cm^3$, etc.). The present inventors have discovered that high cell densities, such as e.g. $10^8$ cell/ml, can be obtained within the context of the present invention at least partially because the cells can grow within the three-dimensional hydrogel structure (both within the hydrogel itself and in cavities/pores within the structure), such that the total cell density in the system is not limited by the presence of a support or carriers onto which the cells adhere but in which they cannot grow (resulting in an inherent density limitation caused by the available surface area and bulk of the support/carrier). Further, the method of the present invention is also not as limited as conventional stirred bioreactors in terms of cellular density as substantial flows of nutrients and gasses can be provided to the cells with a flow of liquid without damaging the cells as much as a stirring system that would be necessary to provide the same nutrients and gasses to a population of cells in a stirred bioreactor. The method may comprise maintaining the cell population until it reaches a predetermined size of cellular aggregates (e.g. spheroids). The predetermined size of cellular aggregates may depend on the cell type, and may for example range between approximately 20 μm in average diameter and approximately 100 μm or approximately 80 μm in average diameter. The present inventors have demonstrated that cellular aggregates with average diameters within these ranges could be obtained using a variety of commonly used production cell lines, using the process of the invention.

The method may comprise culturing the cell population for at least 7 days, at least 10 days, at least 15 days, or at least 20 days. The method may comprise harvesting the cellular product. The step of harvesting the cellular product may be performed one or more times during the culturing step and/or at the end of the culturing step. The present invention advantageously enables the long term maintenance of cells with excellent viability, partially due to the protective nature of the three-dimensional hydrogel structure, its biocompatibility, and the ability to renew the culture medium with limited damage to the cells. The present invention advantageously enables the easy harvesting of cellular products as any cellular product that is present in the culture medium can be harvested by drawing some of the culture medium (preferably with replacement) and any cellular product that is present in the cells (or is the cells themselves) can be harvested by extracting the cellularised structure and separating the cells from the hydrogel matrix.

The method may comprise exchanging at least part of the culture medium at least once during the bioproduction process. The method may comprise providing a flow of culture medium in the bioreactor. The method may comprise a step of washing the cellularised structure. Because the cell population is at least partially embedded in the hydrogel structure, it is relatively easy within the context of the present invention to completely or partially exchange the culture medium in which the cellularised object is located. Indeed, conventional bioprocesses in which the cells are in suspension in the culture medium or on carriers in suspension in the culture medium require the provision of filtering devices in order to remove any culture medium for replacement. Such filtering devices have many issues, such as getting blocked, damaging the cells, resulting in the loss of at least some of the cells through incomplete filtration, etc. Thus, the provision of a system that does not require a filtration device for removal of culture medium from the bioreactor is particularly advantageous. The culture medium may be exchanged fully at regular intervals, such as e.g. every 1, 2, 3, 4 or 5 days. This may be performed through a continuous process, where part of the medium is continuously being replaced, or through a semi-continuous process, where a part of the medium is removed and replaced at regular time intervals. The present inventors have found such medium replacement schemes to advantageously sustain cultures of multiple common production cell lines for long periods of time (such as e.g. up to 20 days or more). The method may comprise maintaining the cellularised structure in a bioreactor comprising one or more inlets and one or more outlets to enable the addition of one or more solutions and the removal of culture medium from the bioreactor. The bioreactor may comprise a flow control device to control the flow of solution/medium in and out of the bioreactor. The one or more inlets and the one or more outlets may be positioned relative to the cellularised object in order to optimise the flow of liquid through the cellularised structure. The flow of solution/medium in and out of the bioreactor may be controlled in order to optimise the flow of liquid through the cellularised structure. Optimising the flow of liquid through the cellularised structure may comprise maximising one or more criteria selected from the homogeneity of the flow through the cellularised structure, the cell viability, the cell density obtainable, etc. This may be performed using a flow modelling software. The provision of a flow of medium ensures that nutrients and gasses are provided to the cells, as well as preventing local accumulation of products and/or by-products of cell growth that may hinder cell growth. Further, a flow of culture medium through and/or around the hydrogel structure advantageously results in lower cell damage compared to stirring in a suspension system. This is believed to be at least in part because of the absence of a rotor and rotary motion, and because the cells are protected by the hydrogel matrix. According to any aspect of the invention, a bioink composition and/or culture medium may further comprise one or more additives, such as e.g. growth factors, nutrients, rheology additives, etc. One or more additives may be chosen specifically for the cell population.

The cellular product may comprise a biological compound or structure secreted or otherwise released by the cells in the cell culture medium. The method may comprise harvesting the cellular product by at least partially removing the culture medium. Harvesting the cellular product may not comprise a cell filtration step. The cellular product may comprise a biological compound or structure that is not released by the cells in the cell culture or wherein the cellular product comprises the cells themselves or a part thereof. The method may comprise harvesting the cellular product by separating the cells from the hydrogel matrix. Harvesting the cellular product may further comprise washing the cells cellularised structure. Separating the cells from the hydrogel matrix may be performed by mechanical separation and/or dissolution of the hydrogel matrix. Harvesting the cellular product may not comprise a trypsination step. The cellular product may comprise one or more biomolecules produced by the cells, viral particles or vectors produced by the cells, the cells themselves or parts thereof such as one or more organelles. The cellular product may be a biopharmaceutical. In a bioproduction process the cellular product needs to be harvested by separating the cellular product from the scaffold (hydrogel structure) and the culture medium. By contrast, in the production of engineered tissues, a biocompatible (often resorbable) scaffold is typically used which can be included together with the engineered tissue for its final use (e.g. implantation). Thus, any method described herein may comprise a step of harvesting the cellular product. Advantageously, because the cells are at least partially embedded and/or contained within the hydrogel structure, the culture medium can be extracted without disrupting the cells, thereby removing the need for cell filtration in order to harvest the product. This makes it easier and more efficient to recover the product, as well as reducing the impact of product harvesting on cell growth and viability. Further, this reduces the problem of cell contamination that is often observed when filtration devices are used (e.g. in conventional stirred bioreactor processes). Additionally, such a harvesting / culture medium exchange process may be performed in a continuous or semi-continuous manner. Where the method comprises at least partially removing the culture medium, the method preferably comprises replacing at least part of the removed culture medium with fresh medium. Harvesting the cellular product may comprise extracting the cellular product from the culture medium. This may be performed using any process known in the art such as e.g. filtration, affinity purification, precipitation, centrifugation, etc. Advantageously, when the cellular product comprises the cells or parts thereof, growth at least partially embedded within the hydrogel matrix structure should enable high cell densities to be reached (typically higher than in e.g. stirred bioreactors or fixed bed bioreactors), thereby resulting in high product yield, without losing the benefits of easy recovery of the cells that is present in stirred bioreactors, and without the negative consequences of the use of a solid support where cells typically have to be separated from the support using treatments such as trypsinisation which can damage the cells. Further, because the cells are at least partially embedded and/or contained within the hydrogel structure, the entire cellularised structure can be easily washed to remove any contaminants, for example prior to harvesting the cells themselves, or to perform a complete change of medium. This is much more difficult to do in a traditional suspension culture, where washing of the cells often requires one or more cell separation steps (such as filtering or centrifugation), which are less efficient and more likely to damage the cells. While washing of the cells on their support may also be possible in a fixed bed reactor, this may cause damage to the cells. By contrast, according to the present invention, because the cells are at least partially embedded and/or contained within the hydrogel structure, any damage associated with washing is reduced. Mechanical separation of the cells from the hydrogel may comprise the use of a mechanical dissociator such as gentleMACS™. Dissolution of the hydrogel matrix may be performed using a chemical dissolution process, such as e.g. using EDTA or sodium citrate. Dissolution of the hydrogel matrix may be performed using an enzymatic process, such as using collagenase A, dispase, pepsin, papain, alginate lyase, trypsin, accutase, tripIE, human platelet lysate, or matrix metalloproteinase(s). Advantageously, an enzymatic process does not use trypsin or an enzyme that is active in conditions that are damaging to the cells (such as e.g. pepsin which is active in strong acidic conditions). For example, an enzymatic process may advantageously use dispase and/or collagenase A. The use of collagenase was shown by the inventors to be associated with high viability and high cell recovery. Dissolution of the hydrogel matrix may be performed by incubating the cellularised object in a solution comprising one or more chemical agents and/or one or more enzymes, then separating the cells from the supernatant for example by centrifugation or through density gradients. The cells may optionally be washed and/or resuspended, for example using a buffer such as PBS, after separation. In particular examples, any of the protocols and solutions described in Example 3 may be used alone or in combinations. The hydrogel matrix may be dissolvable using one or more enzymatic solution(s). The enzymatic solution(s) may comprise one or more of collagenase (e.g. collagenase A), alginate lyase and dispase. The enzymatic solution(s) may not include trypsin. The method may comprise a step of dissolving the hydrogel matrix using one or more enzymatic solutions. The use of a hydrogel matrix that is dissolvable using enzymatic dissolution, particularly ones that do not require trypsinisation was shown by the inventors to be particularly advantageous as this enables high cell recovery with high cell viability. Examples of hydrogel matrices that are dissolvable using enzymatic, chemical and/or mechanical dissolution include matrices comprising alginate, gelatin, fibrin, and/or chitosan, and derivatives thereof.

The cell population and hydrogel matrix structure together may form a cellularised structure and culturing the cell population at least partially embedded in a hydrogel matrix forming a three-dimensional structure may comprise maintaining the cellularised structure in a liquid culture medium comprising at least a proportion of liquid medium in which the cell population has been culture prior to formation of the cellularised structure. The present inventors have discovered that the culture of a cellularised structure in culture medium comprising a proportion of medium in which the cells were culture prior to being included in the hydrogel matrix (also referred to herein as "conditioned medium") either by including the conditioned medium as part of the bioink formulation or as part of the culture medium in which the cellularised object is initially cultured advantageously improves the growth rate and/or cell density achievable using the methods of the disclosure. Where the culture medium is replaced at least one, wholly, partially or continuously during the culturing, the proportion of said conditioned medium may decrease (or be equal to 0) after such replacement. However, the inventors believe that a benefit is still achieved provided that the cell culture in the cellularised structure is initiated in a culture medium comprising conditioned medium. The exact proportion of conditioned medium in new medium is not believed to be crucial. For example, this proportion may be adapted depending on the specific requirements of a bioprocess and/or cell line.

The method may further comprise providing a cellularised structure comprising the cell population and hydrogel matrix by consolidating a composition comprising the cell population and one or more biomaterials capable of forming a hydrogel, by cross-linking one or more of the biomaterials capable of forming a hydrogel. The cross-linking conditions may be set to result in a degree of cross-linking that is compatible with the growth of the cells within the cross-linked matrix. The cross-linking of the biomaterials may include incubation with one or more cross-linking agents (e.g. calcium ions, thrombin, etc). The cross-linking conditions may include an incubation time, incubation temperature, and/or concentration(s) of one or more cross-linking agents. Thus, one of more of the cross-linking conditions may be set to those that result in a maximum cell density after a set time in culture, amongst a set of conditions tested. Thus, the method may further comprise culturing the cell population in a plurality of bioprocesses, wherein the plurality of bioprocesses differs in the consolidation conditions used to obtain the cellularised structure, and determining the consolidation conditions of the plurality of consolidation conditions that result in the highest cell density for the particular cell population and culture conditions after a predetermined culture time.

The method may further comprise providing a cellularised structure comprising the cell population and hydrogel matrix prior to the culturing, wherein the cell density in the cellularised structure is between $1.10^5$ and $1.10^7$ cell / ml of hydrogel matrix or of the composition from which the hydrogel matrix is formed. The present inventors have discovered that these cell densities (also referred to herein as "seeding density") advantageously result in higher growth rates / higher final cell densities when using production cell lines. Further, the present inventors have discovered that the growth rates /final cell densities obtainable with a given seeding density within this range may vary depending on the cell line. For example, when using HEK293 cells the inventors identified that increasing the seeding cell density from $1.10^6$ to $3.10^6$ resulted in a lower final cell density, all other conditions being equal. Thus, the method may comprise a step of determining a seeding cell density that results in the highest final cell density amongst a plurality of seeding cell densities, for a particular cell population and bioprocess.

According to a second aspect, there is provided a system for performing a bioproduction process comprising the production of a cellular product by a cell population, the system including: a bioreactor and one or more cellularised structure(s) comprising a cell population at least partially embedded in a hydrogel matrix forming a three-dimensional structure. The system according to the present aspect may have any of the features described in relation to the preceding aspect. The system according to the present aspect may be configured to implement the method of any embodiment of the first aspect. In particular, the bioreactor and cellularised structure may have any of the features described in relation to the bioreactor and cellularised structure in the preceding aspect. The system may further comprise one of more flow devices to create a flow of medium contained in the bioreactor and in which the cellularised structure is immersed. The system may further comprise one or more support structures within the bioreactor to support the one or more cellularised structures. The system may further comprise one or more monitoring devices such as e.g. a camera, microscope, gas sensor, flow sensor, metabolite sensor, pH sensor, temperature sensor. The system may further comprise one or more supply and/or collection systems for supplying culture medium to the bioreactor and/or collecting culture medium from the bioreactor. The system may further comprise a 3D printing system for depositing a bioink composition from which the cellularised structure is obtained.

Brief Description of the Drawings

Embodiments of the present disclosure will now be described by way of example with reference to the accompanying drawings in which:

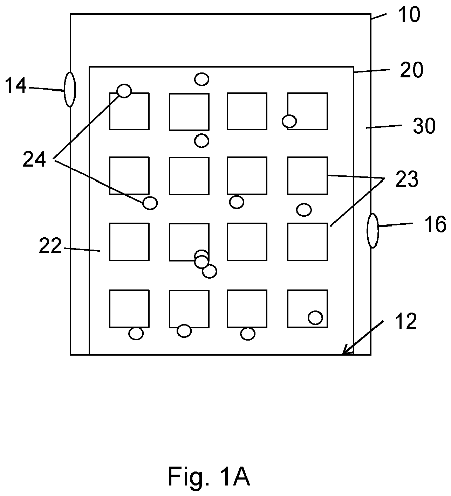

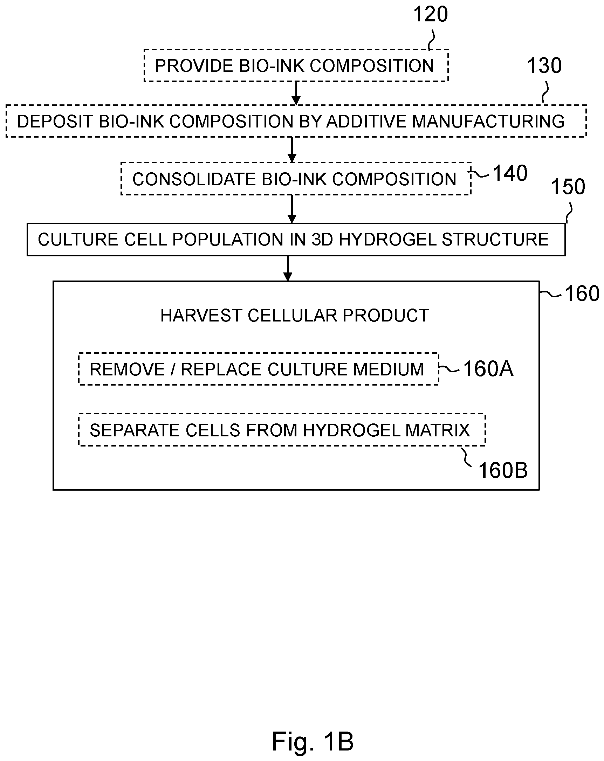

Figure 1 shows (A) components of a generic system for performing a bioprocess as described herein, and (B) a simplified process diagram for a generic bioprocess according to embodiments of the present disclosure;

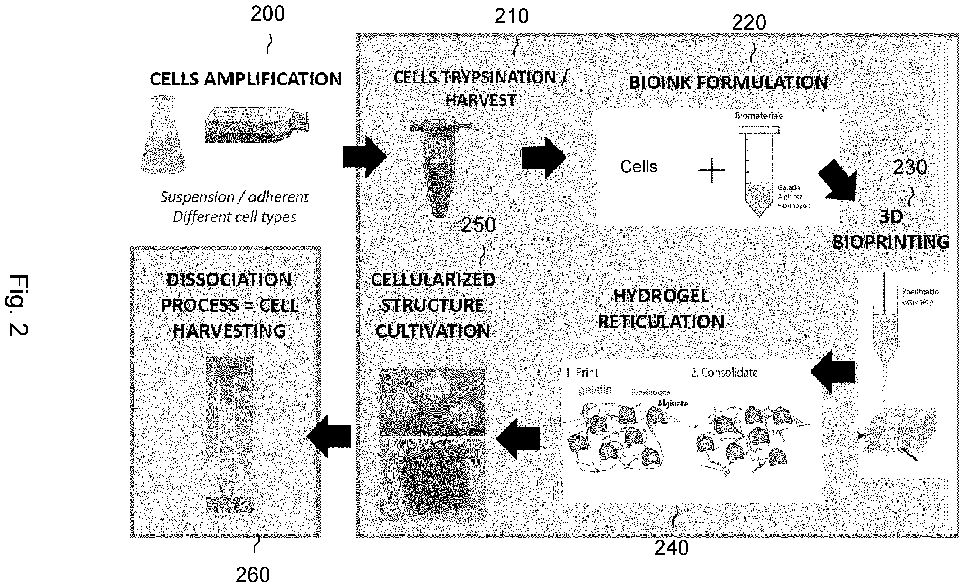

Figure 2 illustrates schematically a process for production of cellular products according to embodiments of the present disclosure;

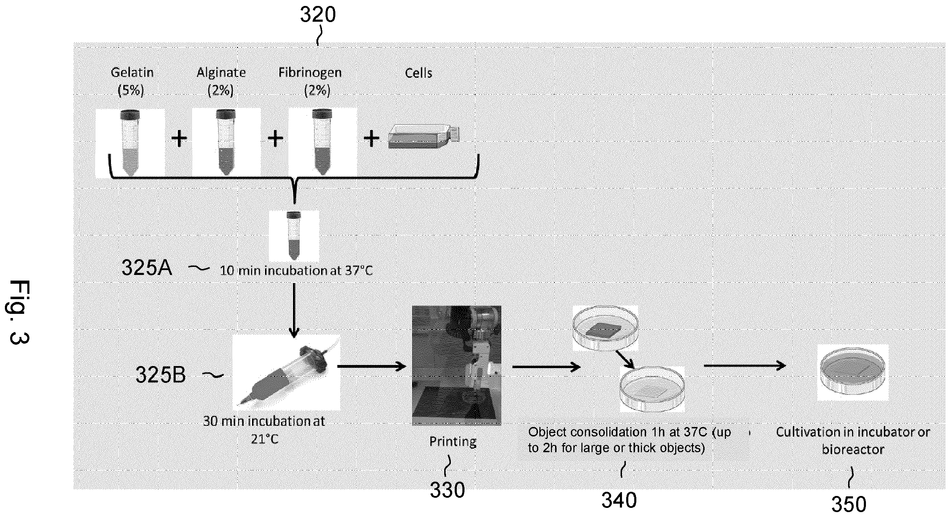

Figure 3 illustrates schematically a process for 3D bioprinting and cellularised structure consolidation according to embodiments of the disclosure;

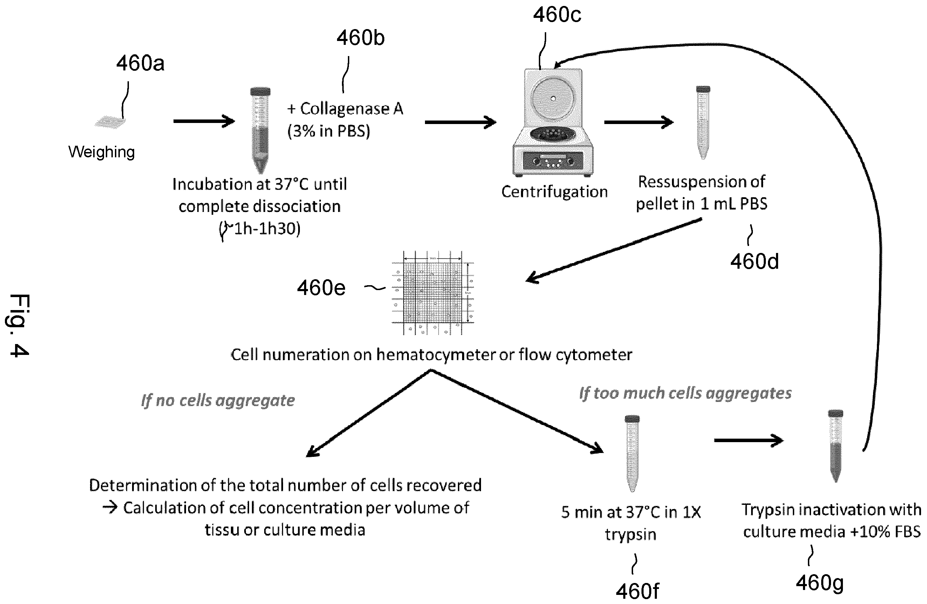

Figure 4 illustrates schematically a process for harvesting of cells from cellularised structures by dissociation, according to embodiments of the disclosure;

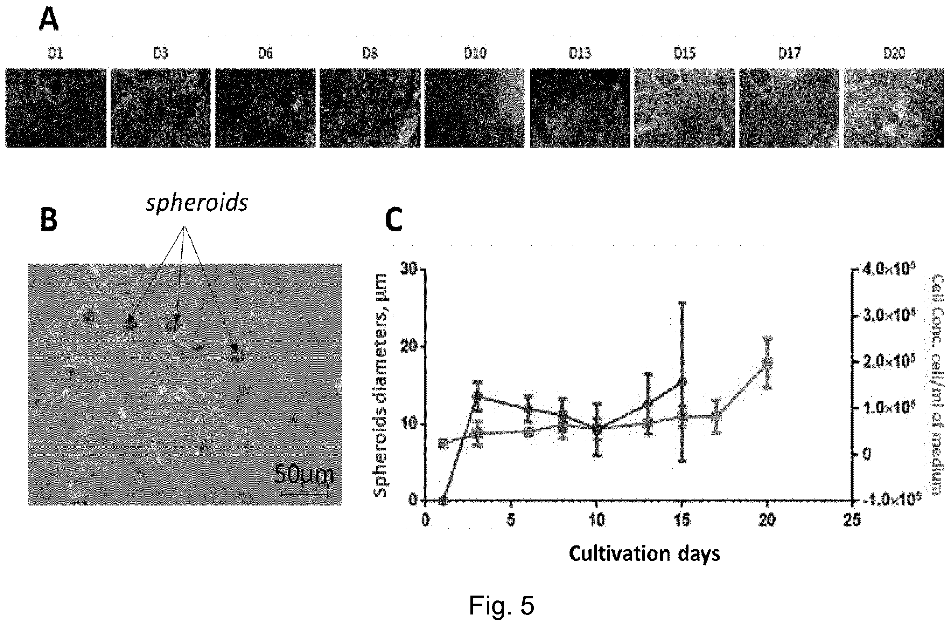

Figure 5 shows the results of an experiment in which the production of CHO cells in 3D bioprinted structures was evaluated. A. Fluorescence microscope images showing the presence and growth of spheroids within the 3D printed biomaterial using calcein staining of live cells. B. Histological cuts of cellularized structures embedded in paraffin and coloured with HE (hematoxylin & eosin) staining after 15 days of culture. C. Growth curve monitoring showing the spheroid diameters (from microscopic observation) and the cell concentration (evaluated by dissociation and counting, dissociation with collagenase 3% in PBS);

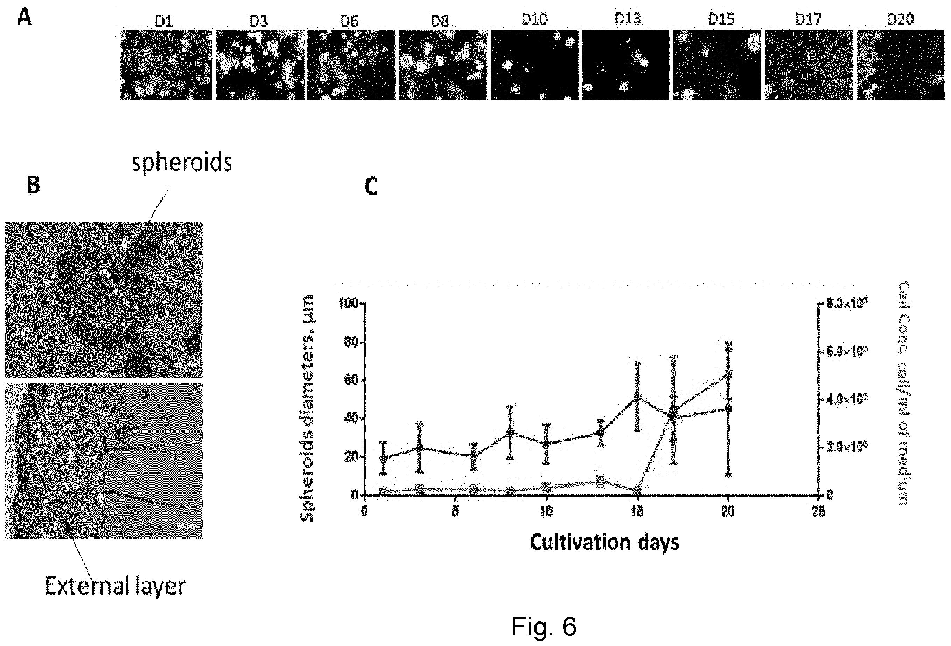

Figure 6 shows the results of an experiment in which the production of HEK cells in 3D bioprinted structures was evaluated. A. Fluorescence microscope images showing the presence and growth of spheroids within the 3D printed biomaterial using calcein staining of live cells. B. Histological cuts of cellularized structures embedded in paraffin and coloured with HE (hematoxylin & eosin) staining after 20 days of culture (cells seeded at $3 \times 10^6$ cell/mL of bioink (eq. to $3.0 \times 10^5$ cell/ mL of medium). The images show the morphology of cells growing as spheroids inside of the bioprinted structures, as well as cells forming a thick layer of cells on the external surface of the bioprinted structures. C. Growth curve monitoring showing the spheroid diameters (from microscopic observation) and the cell concentration (evaluated by dissociation and counting, dissociation with collagenase 3% in PBS);

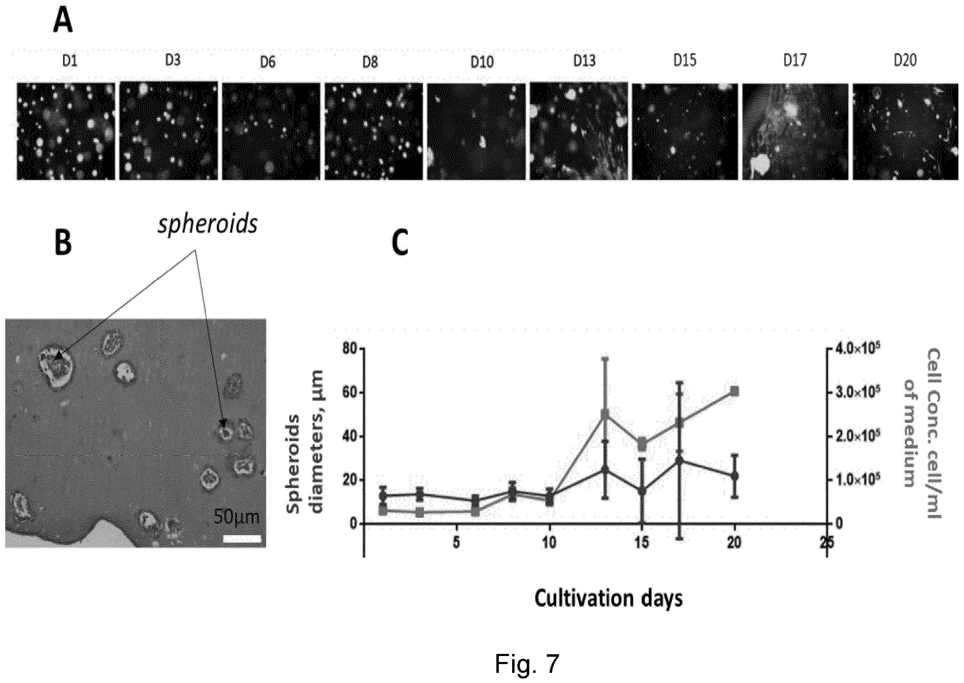

**Figure** 7 shows the results of an experiment in which the production of MDCK cells in 3D bioprinted structures was evaluated. A. Fluorescence microscope images showing the presence and growth of spheroids within the 3D printed biomaterial using calcein staining of live cells. B. Histological cuts of cellularized structures embedded in paraffin and coloured with HE (hematoxylin & eosin) staining after 20 days of culture (cells seeded at $3 \times 10^6$ cell/mL of bioink (eq. to $3.0 \times 10^5$ cell/ mL of medium). C. Growth curve monitoring showing the spheroid diameters (from microscopic observation) and the cell concentration (evaluated by dissociation and counting, dissociation with collagenase 3% in PBS);

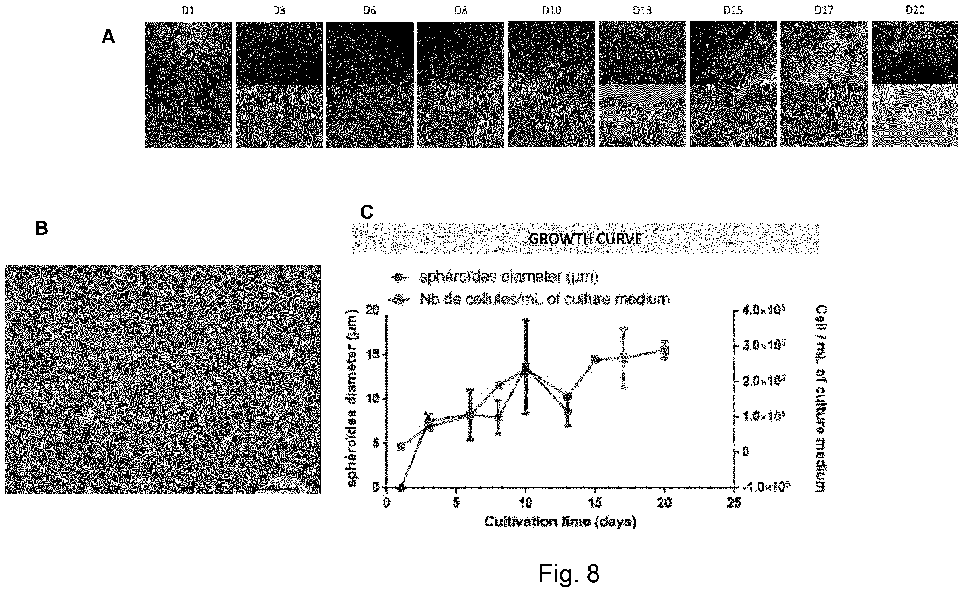

**Figure** 8 shows the results of an experiment in which the production of VERO cells in 3D bioprinted structures was evaluated. A. Fluorescence microscope images showing the presence and growth of spheroids within the 3D printed biomaterial using calcein staining of live cells. B. Histological cuts of cellularized structures embedded in paraffin and coloured with HE (hematoxylin & eosin) staining. C. Growth curve monitoring showing the spheroid diameters (from microscopic observation) and the cell concentration (evaluated by dissociation and counting, dissociation with collagenase 3% in PBS);

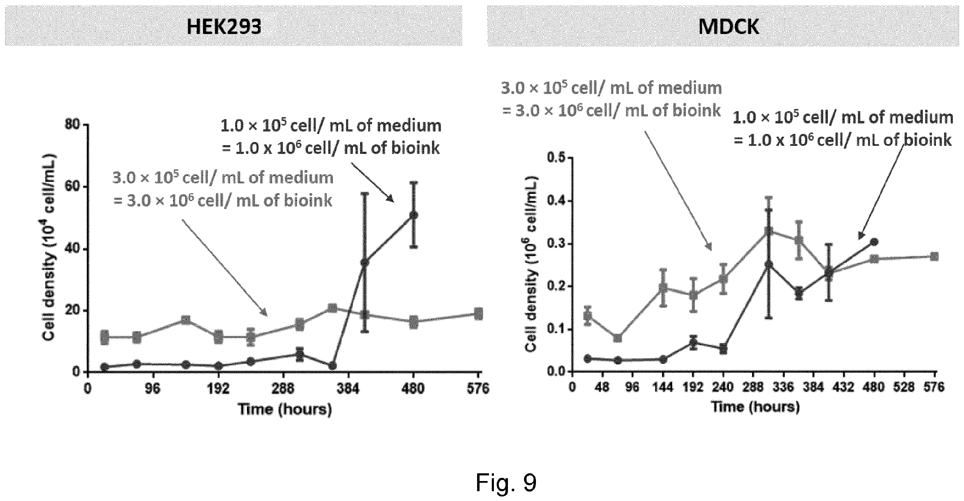

**Figure** 9 shows the results of experiments in which the effect of cell seeding density on growth in a 3D bioprinted structure was investigated for HEK293 cells and MDCK cells;

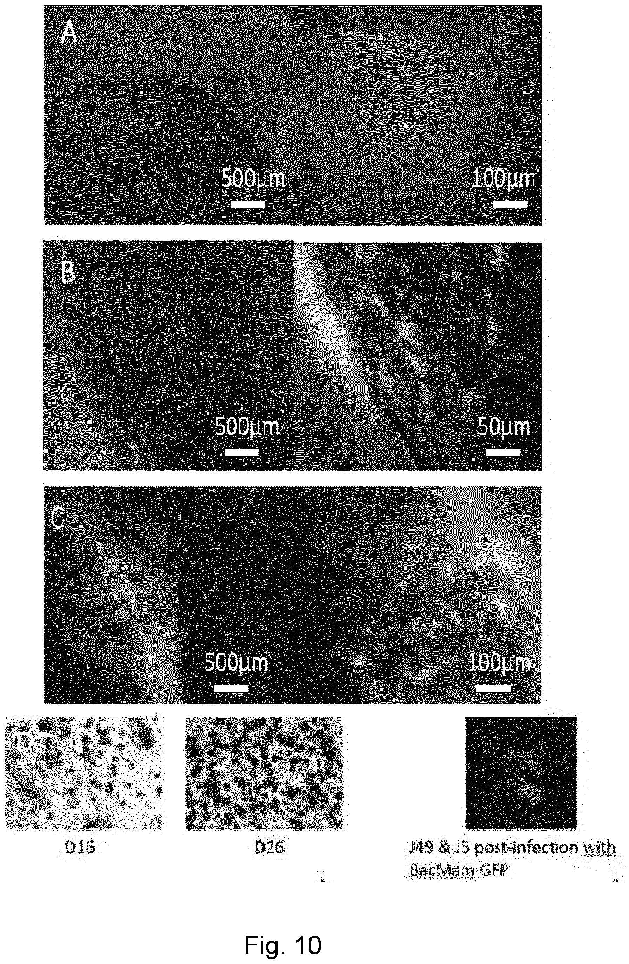

**Figure 10** shows the results of experiments in which the production of a recombinant protein in 3D printed cellularised structures was investigated. A. Fibroblasts infected after 56 days of cultivation by GFP-lentivirus (observation at day 3 post-infection). B. Vero cells infected after 30 days of cultivation by GFP-lentivirus (observation at day 6 post-infection). C. MDCK cells infected after 50 days of cultivation with a BacMam baculovirus-GFP (observation at day 6 post-infection). D. Example of growth and infection process in 3D (MDCK cells: 50 days of growth and 5 days of infection, optical microscope images showing growth and fluorescence microscope showing successful expression of GFP recombinant protein);

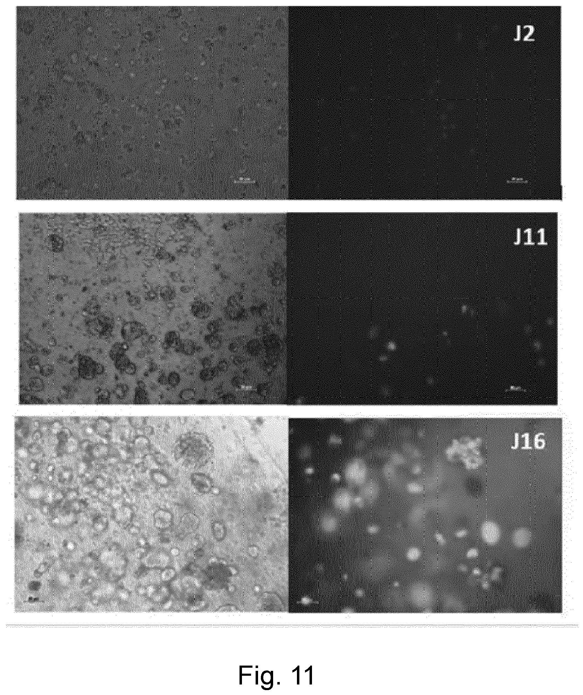

**Figure 11** shows the results of experiments in which the production of a recombinant protein in 3D printed cellularised structures was investigated. HEK293 cells were 3D printed at a cell density of $3 \times 10^6$ cell/ml with GFP-lentivirus. Top row: 2 days growth, middle row: 11 days growth, bottom row: 16 days growth. Optical images showing growth (left column) and fluorescence microscope images showing protein production (right);

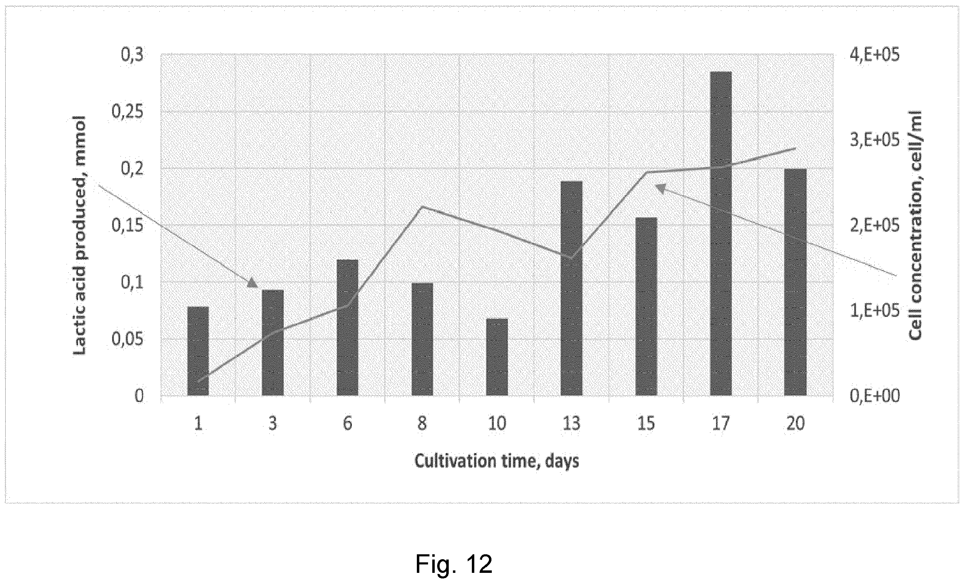

**Figure 12** shows the results of experiments in which the production of a secreted metabolite (lactic acid) in 3D printed cellularised structures was investigated. The cell concentration (HEK293) in the cellularised structure as a function of the time in culture is shown as a single line, and the amount of lactic acid produced as a function of the time in culture (quantified in the culture medium collected every 3 days from the construct culture media without need of prior separation (filtration/ centrifugation) from cellularized construct culture) is shown as a barplot;

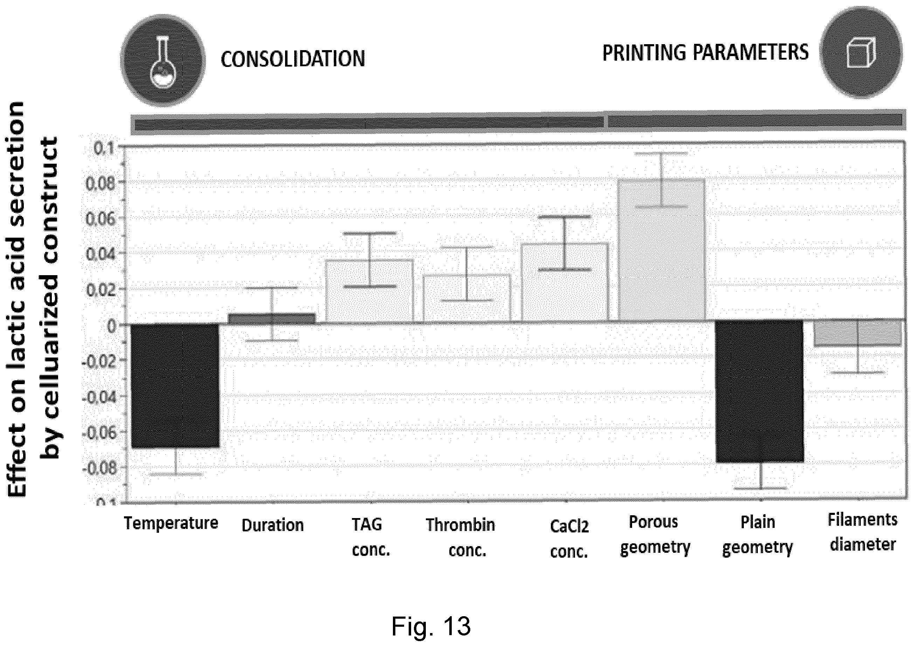

**Figure 13** shows the result of a design of experiment process to evaluate the effect of various consolidation and printing parameters. The effect of the consolidation temperature, duration, concentration of the components of the consolidation solution (transglutaminase (TAG), thrombin and CaCl$_2$), the geometry (porous or no-porous) and the diameter of the printing filament on the amount of lactic acid secreted by the cells in the cellularised structure was investigated;

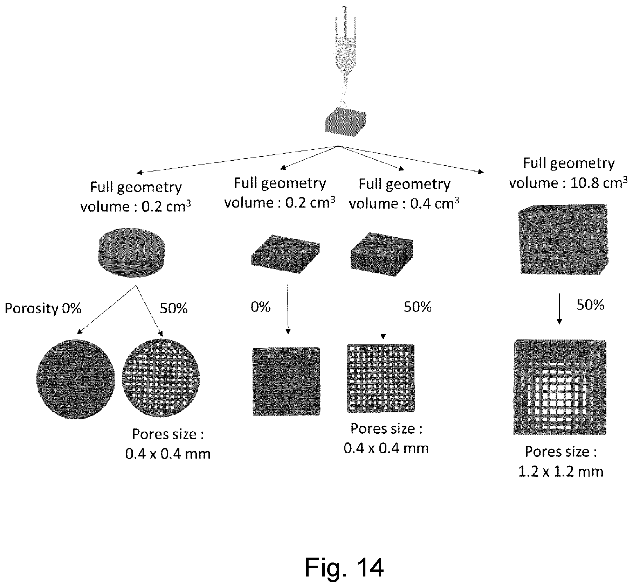

**Figure 14** shows schematically the geometries of deposited objects used in the examples. All geometries were bioprinted. The deposited objects had different volumes (indicated as "full geometry volume") and different porosities (indicated in %, with the pore sizes indicated). All pores were channels with a square cross section as indicated;

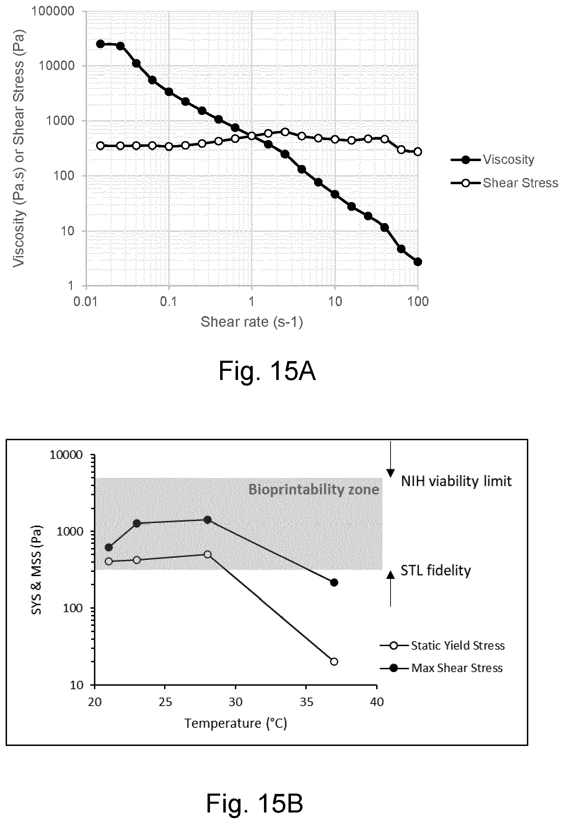

**Figure 15** shows exemplary results for a process of determining the rheological properties of a bioink composition and printing conditions associated with rheological properties suitable for printing. A. Example reference rheogram for a bioink composition according to the disclosure, with rheological properties suitable for printing (maintain shape fidelity and cell viability). B. Representation of the bioprintability zone for a bioink formulation with rheological properties as in A. Here different rheological behavior were tested with regards to the shape fidelity of the bioprinted object (STL fidelity) and with regards to the viability of cells after the microextrusion process (NIH fibroblast cell viability). The shaded zone represents the shear stress (MSS) and static yield stress (SYS) that cells can handle.

Where the figures laid out herein illustrate embodiments of the present invention, these should not be construed as limiting to the scope of the invention. Where appropriate, like reference numerals will be used in different figures to relate to the same structural features of the illustrated embodiments.

Detailed Description

Specific embodiments of the invention will be described below with reference to the figures.

As used herein, the term "bioprocess" (also referred to herein as "biomanufacturing process") refers to a process where biological components such as cells, parts thereof such as organelles or multicellular structures such as organoids or spheroids are maintained in a liquid medium (typically either in suspension or supported on a scaffold) in an artificial environment such as a bioreactor, so that they can perform a function (such as e.g. the production of a biomaterial). According to the present invention, a cell population is maintained at least partially embedded in a hydrogel matrix forming a three-dimensional structure (referred to herein as "cellularised object" or "deposited object"). The cell population may be at least partially embedded in the hydrogel matrix in that at least a portion of the cell population grows within the hydrogel matrix as opposed to on the surface thereof. Typically, the cells growing within the hydrogel matrix grow as spheroids. Part of the cell population may also grow on the surface of the hydrogel matrix. This may be removed if desired, for example, if the cells that grow on the surface of the cellularised object interfere with the control of the flow through the structure. A bioreactor is a device in which a biological component can be maintained in controlled environmental and operating conditions (e.g. pH, temperature, pressure nutrient supply, and waste removal). A bioreactor typically comprises at least a vessel suitable for holding a liquid culture medium. Thus, as used herein, the term "bioreactor" does not necessarily refer to a standard stirred tank for suspension cultures, also such vessels are encompassed by the term. The configuration, shape and/or volume of the bioreactor may depend in the bioprocess. The vessel itself may be 3D printed. The vessel may be a single use vessel, or a reusable vessel. The vessel may be made primarily of stainless steel, plastic or any other material known in the art for use in industrial bioprocesses. For example, in embodiments comprising one or more cellularised objects (i.e. a three dimensional structure comprising a hydrogel matrix and cells at least partially embedded in the hydrogel matrix) cultured in suspension, a stirred tank bioreactor may be used. The volume of the tank may be determined based on the requirement of the bioprocess, such as e.g. the desired number of cells at the end of the bioprocess. As another example, the volume of the vessel may be determined based on the size of the cellularised object(s) and/or the configuration of the supports (in embodiments comprising one or more cellularised objects cultured on a support in a vessel), for example to ensure that the cellularised object(s) is/are fully immersed in culture medium and/or to obtain a ratio of volume of culture medium to volume of cellularised object within a desired range and/or to obtain number of cells in the cellularised object per volume of culture medium within a desired range. For example, it may be advantageous for the ratio of volume of culture medium to volume of the cellularised object(s) / volume of the bioink used to obtain the cellularised object(s) to be between 5 and 50, preferably below 50, such as e.g. 10, 20, 30 or 40 (e.g. 10 ml of culture medium for every $cm^3$ of cellularised object / deposited bioink). As another example, it may be advantageous for the number of cells per ml of culture medium in the vessel to be at least $10^4$ cell/ml (such as e.g. between 1.0E+04 cell/ml and 1.0E+08 cell/ml, preferably at least 1.0E+05 cell/ml) at the start of the culture (i.e. seeding density). It may further be advantageous for the number of cells per ml of bioink used to make the cellularised object(s) to be at least $10^6$ cells/ml. The present inventors have identified that such cell densities lead to better growth rates and ultimately higher final cell numbers. Within the context of the present invention, the bioprocess comprises a cell culture. A bioprocess typically results in a product, which can include biomass and/or one or more compounds that are produced as a result of the activity of the biological components. A bioreactor can be a single use vessel or a reusable vessel in which a liquid medium suitable for carrying out a bioprocess can be contained. The present invention is applicable to any type of bioreactor and in particular to any vendor and any scale of bioreactor from benchtop systems to manufacturing scale systems.

A cell culture refers to a bioprocess whereby live cells are maintained in an artificial environment such as a bioreactor. The methods and systems described herein are applicable to bioprocesses that use any types of cells that can be maintained in culture, whether eukaryotic or prokaryotic. Preferably, the cells are "production cells". In embodiments, the cells are from a "production cell line". A cell line is a population of immortal cells maintained in artificial (culture) conditions. Production cells are cells from cell populations developed for the production of biomaterials and that typically have higher growth rates and/or protein expression rates and/or metabolic rates than primary cells of the same origin. Production cells are typically from production cell lines. Production cells may be derived from primary cells, such as e.g. mesenchymal stem cells derived from bone marrow. Such cells may have good capacity for in vitro expansion and may be used for the production of cells of particular cell types and/or for production of proteins of interest. The cells may be animal cells, such as mammalian or insect cells. Preferably, the cells are animal cells that are not insect cells. In embodiments, the cells are mammalian cells. In embodiments, the cells are all from the same cell type, preferably the same cell line. The cells may be selected from MDCK cells (Madin-Darby Canine Kidney cells, which are epithelial cells from dog kidney), VERO cells (epithelial kidney cells from green monkey), AGE.CR1™ (ProbioGen, Muscovy duck retina cells designed for virus-based vaccine production), PRE.C6 (a cell line derived from human embryonic retinal cells), EB.14 (a chicken embryonic stem cell line), EP.66™ (a duck embryo-derived cell line), HEK293 (a human embryonic kidney cell line), BHK21 (a baby hamster kidney cell line), CHO (Chinese hamster ovary cell lines), NS0 (a murine myeloma cell line), Sp2 (a cell line derived from the fusion of a BALB/c mouse spleen cell and a mouse myeloma cell line), Sf9 (a clonal isolate of *Spodoptera frugiperda* Sf21 cells), SF21 (a cell line derived from ovarian *Spodoptera frugiperda* cells), MRC-5 (human normal lungs fibroblasts), WI-38 (human normal lungs fibroblasts), CEF (chicken embryo fibroblasts), hybridomas (murine antibody producing B-cells fused with myeloma cells). In embodiments, the cells are selected from VERO cells, CHO cells, MDCK cells, and HEK293 cells. Successful growth of each of these cell lines in the context described herein has been demonstrated by the inventors.

A product of a bioprocess (also referred to herein as "cellular product", "biomaterial" or "target biologic") may include a metabolite, a cell or a part thereof (such as e.g. an organelle), a virus or viral particle, a desired protein (e.g. an antibody, an immunoglobulin, any recombinant protein), a toxin, one or more by-products, or any other type of molecule manufactured using a bioprocess. There may be more than one biomaterial of interest. For example, the cells may secrete a metabolite of interest that may be harvested from the culture medium, and the cells themselves may also represent a product of interest that may be harvested at the end of the culture. As another example, the cells may produce a plurality of proteins and/or metabolites of interests, some of which may be secreted in the culture medium (and e.g. harvested during the culture), and some of which may be retained in the cells (and harvested at the end of the culture, e.g. through cell lysis and product purification). The cellular product may be a biopharmaceutical, also referred to as "biologics". The present invention is particularly advantageous in this context as biopharmaceuticals are typically very high value added products (due to the complexity of the production process, leading to small changes in productivity having very high impacts), and the products have high quality requirements (e.g. high purity requirements).

The invention relates at least in part to the use of a 3D hydrogel structure in which cells are embedded, and in the culture of said cells in such a proliferative hydrogel matrix. The 3D hydrogel structure is typically obtained through a two-step process that involves forming a composition comprising biomaterials capable of forming a hydrogel into a 3D shape (also referred to as "deposited object" or "cellularised structure/object") and consolidating the object so that it maintains its shape in culture. The step of forming the composition can be performed by molding (casting, etc.), or by 3D printing. The step of forming the composition is preferably performed by 3D printing. The term "3D printing" (also referred to as "additive manufacturing") refers to the creation of a 3D structure by computer controlled deposition of a material. Within the context of the present invention, the material is typically a bioink, as described below. 3D printing involves the controlled deposition of material into a three-dimensional shape. 3D printing typically follows a computer-aided design (CAD) model. CAD models can be stored as STL (stereolithography file format) files or AMF (Additive Manufacturing File format) files. 3D printing may for example be achieved by extrusion (for example, using a syringe with a piston or screw) or by ink-jet (by projection of ink droplets). Within the context of the present disclosure, 3D printing is typically achieved by extrusion, particularly pneumatic extrusion. However, 3D printing by inkjet is also possible and explicitly envisaged. For example, a bioink may be loaded into an extrusion syringe that is mounted on a bio-printer. The syringe may be equipped with a pusher to control the flow of bioink. As described further below, the bioink may be in a flowable consistency during printing, and may be rapidly solidified when deposited by the 3D printer. This ensures that the printed structure (also referred to as "deposited object" or "cellularised structure/object") maintains its 3D shape. Once solidified, the bioink may be further consolidated by polymerisation, as will be explained further below. The deposited object is typically formed by progressive addition of material in the form of beads of material or a filament of material. The deposited object may be formed from filaments of material that are between 200 and 800 microns in diameter. The present inventors have found such dimensions to be particularly suitable for the culture of cells at high density. Without wishing to be bound by theory, the inventors believe that this may be due to higher amounts of cell growth occurring on the surface of filaments than within filaments, such that a balance can be obtained between stability (and protective nature) of the structure and growth of the cells within the above-mentioned range of dimensions. In particular, the nutriment diffusion may not be limiting within such a range of filament diameter, and thus cells can preferentially grow at and close to the surface of the filaments. Preferably, the deposited object has an internal three-dimensional structure. In other words, the deposited object is preferably not a solid form such as a solid cube. This may also be referred to as the deposited object comprising cavities or pores. Thus, the deposited object may be referred to as having a certain porosity. The porosity of a deposited object may refer to the ratio of the volume of the pores to the total external volume of the deposited object. When using 3D printing to form a cellularised object, the porosity of the cellularised object can advantageously be tightly controlled through control of the deposited shape. Further, this can be easily varied by changing the shape that is being printed. In embodiments, the pores can have a cross section diameter between 500 and 2000 $\mu$m, where the diameter of a pore refers to the diameter of the smallest circle that fits the entire cross-section of the pore. The pores may be in the form of channels with a constant cross section. For example, a square cross section may be used. Pores with a square cross section may for example have a cross section with sides between 400 and 1200 $\mu$m (leading to a diameter between approximately 566 and 1697 $\mu$m). Examples of suitable geometries are shown on Figure 14. The total volume of the deposited object refers to the external volume of the deposited object. Advantageously, when using 3D printing the total volume of the deposited object may be varied, as well as the external shape of the object and its internal structure, to suit a particular application (such as e.g. a particular scale of production, a particular cell type, a particular bioreactor, etc.). This advantageously may not require changing the material used and/or the hardware used for printing. Thus, a variety of shapes and dimensions for the deposited object are envisaged. In embodiments, the deposited object has dimensions such that the total volume of the deposited object is in the order of $cm^3$ (e.g. 1 to 10 $cm^3$) or $dm^3$ (e.g. 1 to 10 $dm^3$). For example, the three-dimensional structure may have been obtained by depositing between 1 ml and 1l of bioink, between 1 ml and 500 ml of bioink or between 1 ml and 250 ml of bioink. Deposited objects with dimensions in these ranges may advantageously enable to obtain cell densities (in number of cells per ml of culture medium in the vessel) similar or higher to those achievable in cell production processes in perfused cell culture. For example, standard perfusion processes are commonly operated at 20-35 $\times 10^6$ cells/ml in 4l cultivation vessels. According to the present invention, cells are cultured at least partially embedded within a three-dimensional structure comprising a hydrogel matrix, which provides a protection to the cells and enables to maintain the cells inside of the vessel without the use of a separation process. This can enable even higher cell densities to be reached than is possible in a standard perfusion process where the cells are cultured in suspension. For example, cell densities of $10^7$ cells/ml (volume of bioink), $10^8$ cells/ml (volume of bioink) or more may be reached (such as e.g. up to $10^9$ cell/ml or $10^{10}$ cell/ml of bioink), depending at least in part on the porosity of the object and the size of the cells cultured. For example, assuming that cells have an average diameter between 7.15 to 9.14 $\mu m$ (as measured for CHO cells by Sakhr et al., Int J Mol Sci. 2021 Apr; 22(7): 3290, 2021), and assuming a porous deposited object with 50% porosity, the maximal cell density that could be reached may be of the order of $7.5 \times 10^9$ cell/$cm^3$ (or $7.5 \times 10^9$ cell/ml) of bioink. Thus, the same number of cells as typically cultured in a 4l cultivation vessel ($35 \times 10^6$ cell/ml) can be obtained using a deposited object that was made from 250 ml of bioink. As explained above, such a deposited object may be cultured in a vessel comprising a ratio of culture medium to volume of deposited object (volume of bioink) between 5 and 50, i.e. in this case a vessel of between 1.25 l and 12.5 l. Such three dimensional structures may be supported on a surface. For example, the deposited object may be a cube or parallelepiped. In embodiments, the deposited object may have a here-dimensional lattice structure. Preferably, the lattice structure is a regular lattice structure. For example, the deposited object may have a regular three-dimensional cubic lattice structure. This advantageously results in a simple structure that is relatively easy to print, enables a homogeneous flow of liquid through the structure and comprises pores of homogeneous sizes, thereby increasing the likelihood that the cell culture within the structure will be homogeneous. In embodiments, the deposited object has a plurality of porous external walls (also referred to as "sides" or "faces") and a plurality of solid external walls. This may enable a controlled flow of liquid (such as e.g. culture medium) through the deposited object, as a flow can only enter and exit the deposited object through the porous external walls. For example, the deposited object may have two substantially parallel porous external walls, enabling a parallel flow through each of the two porous external walls. The other walls may be solid in order to guide the flow through a first porous external wall, through the object and through a second porous external wall. Alternatively, the three-dimensional structure may have dimensions such that the total (external) volume of the three-dimensional structure is in the order of $\mu m^3$ (e.g. structures with an external diameter of between 50 $\mu m$ and 500 $\mu m$, or with an external volume of $65.10^3$ to $65.10^6$ $\mu m^3$ or 0.065 $mm^3$) to $mm^3$ (e.g. 1 to 1000 $mm^3$). Such three dimensional structures may be cultured in suspension. Such three dimensional structures may be deposited as droplets rather than filaments.

The deposited object may be incubated in a vessel also called a "bioreactor". The bioreactor may be equipped with one or more fluid inlet(s) and one or more fluid outlet(s). The fluid inlets and outlets may enable the provision of nutrients, gasses etc. to the cells in the deposited structure, by creating a flow of medium through the deposited object. The fluid inlet(s) and outlet(s) may respectively be connected to a supply tank and collection system. The flow at the inlet(s) and/or outlet(s) may be constant or variable during the bioprocess, whether in composition or in volumetric flow. For example, the flow of liquid through the bioreactor (assuming that the volume of liquid flowing into the bioreactor is equal to the flow volume of liquid flowing out of the bioreactor) may be increased one or more times during the bioprocess, to support the growing number of cells. The deposited object may be positioned relative to a flow inlet and outlet of the bioreactor in which the deposited object is incubated so as to optimise the characteristics (e.g. direction, speed etc.) of the flow of liquid through the deposited object. The flow of liquid at the inlet(s) and outlet(s) may be controlled for example to maintain the fluid velocity at the inlet/outlet within a predetermined range. This range may be adapted for each inlet/outlet in order to optimise the flow of liquid through the deposited structure, for example to ensure adequate supply of nutrients / gasses while maintaining cell viability by controlling mechanical stress to the cells within the deposited structure.

As used herein, the terms "computer system" of "computer device" includes the hardware, software and data storage devices for embodying a system or carrying out a computer implemented method, such as e.g. for printing a 3D object as part of a 3D printing system. For example, a computer system may comprise one or more processing units such as a central processing unit (CPU) and/or a graphical processing unit (GPU), input means, output means and data storage, which may be embodied as one or more connected computing devices. Preferably the computer system has a display or comprises a computing device that has a display to provide a visual output display (for example in the design of the business process). The data storage may comprise RAM, disk drives or other computer readable media. The computer system may include a plurality of computing devices connected by a network and able to communicate with each other over that network. For example, a computer system may be implemented as a cloud computer. The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

The term "hydrogel" refers to a crosslinked hydrophilic polymer that does not dissolve in water. Within the context of the present invention, a hydrogel may be obtained by consolidation (also referred to as "polymerisation" or "cross linking") of a bioink. A bioink is a composition comprising one or more biomaterials that can form a hydrogel in appropriate polymerisation condition, and one or more cells. Suitably, a bioink as described in US 2019/002836A1 may be used, the entire content of which is incorporated herein by reference. A biomaterial is a biocompatible material, i.e. a material that is compatible with the maintenance of living cells. In embodiments, the one or more biomaterials that can form a hydrogel comprise alginic acid or a sodium salt thereof also referred to as "sodium alginate" or "alginate" (a naturally occurring polysaccharide which can form a polymer called "alginate" in the presence of e.g. Calcium ions, a process also called "gelation"), fibrinogen (a naturally occurring glycoprotein complex which can form a polymer called "fibrin" by enzymatic conversion by thrombin, a process also referred to as "coagulation"), and/or gelatin (a peptide composition that can form a hydrogel when hydrated and maintained at temperatures below gelling point). In embodiments, the one or more biomaterials that can form a hydrogel comprise chitosan. For example, the one or more biomaterials that can form a hydrogel may comprise chitosan, alginate and fibrinogen. As another example, chitosan may be the only biomaterial capable of forming a hydrogel that is used in the bioink. At least one of the biomaterials is such that the polymerised object is stable in the desired cell culture conditions. For example, when the cell culture conditions comprise a temperature T, at least one of the biomaterials is such that it remained polymerised at temperature T. As another example, when the cell culture conditions comprise a concentration of one or more components (such as e.g. ions) within a certain range, at least one of the biomaterials is such that it remains polymerised in media having said composition. As another example, when the cell culture conditions require a pH within a certain range, at least one of the biomaterials is such that it remains polymerised in media having a pH within said range. It is advantageous for at least one of the biomaterials used to be such that polymerisation is not reversible, in order to ensure the stability of the hydrogel in culture conditions. For example, the coagulation of fibrinogen is irreversible, whereas the gelling of alginic acid can be at least partially reversed through removal of calcium ions, and the gel formed by gelatin can be melted by reheating. Other biocompatible materials that can form a hydrogel may be used. The one or more biomaterials may comprise one or more biomaterials that can form a hydrogel, and one or more additional components or additives. The one or more additional components may provide one or more desired properties to the bioink and/or the hydrogel. In embodiments, at least one of the biomaterials and/or one of the one or more additional components ensures that the bioink has desired rheological properties (such as e.g. a desired consistency and/or viscosity) prior to and during deposition. For example, the composition may comprise one or more components that ensure that the bioink can maintain its shape at least temporarily, e.g. during printing, prior to the irreversible consolidation of the biomaterials into a hydrogel. The rheological properties of the bioink and the associated conditions for printing that enable the bioink to maintain its shape at least temporarily while being compatible with cell viability may be assessed for example as described in the examples. The one or more additional components may also be biomaterials. For example, the bioink may comprise a gelling agent, such as e.g. gelatin. A gelling agent such as e.g. gelatin may be used to maintain the shape of the bioink for example during deposition, by cooling the deposited bioink. The bioink may then be treated with a polymerisation solution (or a plurality of solutions) in order to cause the other biomaterials to form a hydrogel. When the bioink comprises alginic acid, the polymerisation solution(s) may comprise calcium ions. When the bioink comprises fibrinogen, the polymerisation solution(s) may comprise thrombin. In embodiments, the polymerisation solution(s) may comprise transglutaminase (TAG). The present inventors have identified that it is advantageous for the consolidation solution to comprise transglutaminase as this may enhance the long term stability of transforming agent (in particular DNA, such as e.g. plasmid DNA) in the deposited object. It is advantageous for the gelling agent to have a gel point (also referred to as "transition point" or "gel melting temperature") that is compatible with cell viability, i.e. that is such that there exists a range of temperatures compatible with cell viability that is below the gel point of the gelling agent, and a range of temperatures compatible with cell viability that is above the gel point of the gelling agent. For example, the gelling agent may have a melting temperature below 35°C. For example, a gelatin with a gel point between 27 and 32 °C (such as e.g. between 28 and 30°C) may be used. Thus, the bioink may be deposited while the composition is a temperature that is above the gelling point of the composition (such as e.g. between 28 and 37°C), or slightly below the gelling point of the composition (such as e.g. between 21 and 28°C), such that the bioink is fluid, and rapidly cooled at / after deposition to a temperature that is below the gelling point of the composition after deposition (such as e.g. between 0 and 20°C) such that the bioink gels. This may be achieved for example by depositing the bioink onto a cooled substrate. The temperature of the cooled substrate and/or the environment in which the bioink is deposited may be adjusted depending on the temperature of the bioink and the rate of deposition, in order to ensure that the deposited object maintains its shape. In embodiments, the bioink may be deposited while the composition is a temperature between 21 and 28°C. In embodiments, the consolidation of the other biomaterials is performed at a temperate above the gelling point of the gelling agent. This may re-dissolve at least part of the gelling agent which can then be removed, when the other biomaterials have been polymerised. This may further be advantageous in optimising the activity of any enzyme that is active during consolidation. For example, a temperature of approximately 37°C is advantageous for consolidation with thrombin. The bioink may comprise a first solution comprising between 5% and 40% w/v (weight by volume) of gelatin (such as e.g. approximately 20% w/v of gelatin), a second solution comprising between 1% and 12% w/v of alginate (such as e.g. approximately 4% w/v of alginate), and a third solution comprising between 1% and 15% w/v of fibrinogen (such as e.g. approximately 8% by mass fibrinogen). The first solution may represent 35 to 65% by volume of the bioink (such as e.g. approximately 50% by volume). The second solution may represent 15 to 35% (such as e.g. approximately 25%) by volume of the bioink. The third solution may represent 15 to 35% (such as e.g. approximately 25%) by volume of the bioink. Thus, the bioink may comprise between 1.75% and 26% w/v of gelatin, between 0.15% and 4.2% w/v of alginate (e.g. sodium alginate), and between 0.15% and 5.25% w/v of fibrinogen. For example, the bioink may comprise approximately 5% w/v gelatin, 2% w/v alginate and 2% w/v fibrinogen. The bioink has an osmotic pressure compatible with cell viability. Thus, the bioink may comprise a solution that preserves the osmotic pressure of the cells in the bioink. For example, the bioink may have a NaCl content between 0.2% and 5% w/v. Preferably, the solution that preserves the osmotic pressure of the cells in the bioink is a nutritive solution. For example, the bioink may comprise a culture medium solution. A culture medium (also referred to as "growth medium") solution is a solution designed to support the growth of a population of cells. Such a solution is typically sterile, and comprises substances required for the growth of cells. Examples of culture media are known in the art and include, DMEM, RPMI 1640, MEM, F-12 K, etc. The growth medium may be a calcium free growth medium. For example it may be calcium free DMEM, herein referred to as DMEM(-). In embodiments, the bioink composition comprises one or more solutions (as explained above), each of which comprises one or more biomaterials and/or cells, in a nutritive solution that preserves the osmotic pressure of the cells (such as e.g. a culture medium, e.g. DMEM(-)). For example, the first solution mentioned above may comprise between 5% and 40% w/v (weight by volume) of gelatin in culture medium (e.g. DMEM(-)). Similarly, the second solution may comprise between 1% and 12% w/v of alginate in culture medium. The third solution may comprise between 1% and 15% w/v of fibrinogen in culture medium. The precise culture medium used may depend on the cells to be cultured. The bioink may be consolidated using a polymerisation solution. When the bioink comprises alginate and fibrinogen, the polymerisation solution may comprise calcium ions and thrombin. The polymerisation solution may comprise between 1% and 5% w/v of a calcium salt (such as e.g. 3% w/v $CaCl_2$) and between 2 and 40 U/mL (such as e.g. approximately 10 U/mL) of thrombin. For consolidation, the deposited bioink may be incubated in the polymerisation solution for a predetermined period of time (such as e.g. 1 hour), for example by immersion of the deposited object in the polymerisation solution. Immersion of the deposited object in the polymerisation solution may advantageously result in a homogeneous polymerisation of the biomaterials. The bioink composition may be prepared for use from one or more stable aqueous solutions (such as e.g. the first, second and third solutions described above), and a suspension or pellets of cells. When a suspension of cells is used, the suspension may be in a culture medium. When alginate is used as one of the biomaterials, the suspension of cells (and every other solution used to prepare the bioink) is preferably calcium free. For example, a calcium free culture medium such as DMEM(-) may be used to suspend the cells prior to mixing with the other components of the bioink. When the deposited object has been polymerised, the cells within the object may then be cultured in this environment. The cells may therefore be contained within and protected by a three-dimensional network formed by the consolidated biomaterials themselves, and any three-dimensional structure formed by the shape of the deposited object.

A deposited object according to the invention is preferably maintained within a liquid culture medium, wherein the deposited object is fully immersed within the medium. The culture medium (also referred to simply as "medium") can be any medium that is suitable for the culture of cells as described herein, in conventional stirred bioreactors. In embodiments, the culture medium is a serum free culture medium, or a chemically defined culture medium. In embodiments, the culture medium is a calcium free culture medium. In embodiments, the culture medium does not comprise additives used to protect the cells from hydrodynamic damage, such as detergents. The culture medium may be the same culture medium used to prepare the bioink.

**Figure 1A** illustrates schematically a system according to the disclosure. The system comprises a vessel (also referred to as "bioreactor") 10, in which one or more deposited objects (also referred to as "cellularised structure") 20 is/are maintained in a culture medium 30. In the illustrated embodiment the culture medium 30 completely surrounds the deposited object(s). In the illustrated embodiment, the deposited object(s) is/are each supported by an internal surface 12 of the bioreactor 10. In other embodiments, the deposited object(s) may be in suspension in the culture medium 30. In such embodiments, an agitation system (e.g. a stirring system) may be provided. In the illustrated embodiment, a single deposited object is shown, supported by an internal surface 12 of the bioreactor 10. However, in alternative embodiments a plurality of deposited objects may be supported by the same or different internal surfaces of the bioreactor. The internal surface(s) may be an internal wall that is part of the outer structure of the bioreactor, or may be an additional wall that is provided at least in part for the purpose of supporting the one or more deposited objects. For example, the bioreactor may be provided with one or more shelves each supporting one or more deposited objects. Optionally, the bioreactor 10 has one or more inlets 14 and one or more outlets 16 to allow the flow of culture medium 30 into and out of the bioreactor 10. In other words, the system may be a perfusion system (i.e. the vessel may be a perfusion bioreactor). A perfusion bioreactor is a cell culture vessel in which cells are retained in the vessel while a continuous inflow of fresh culture medium is provided and a continuous outflow of spend medium is extracted. The present inventors have discovered that a perfusion system enabled more efficient growth of cells at least partially embedded within a 3D hydrogel. The inlets 14 may be connected to a supply system (not shown), which may include e.g. a tank. The tank may be a stirred tank. The supply system may supply fluid (e.g. fresh culture medium) to one or more bioreactors 10. The outlets 16 may be connected to a collection system (not shown), which may include one or more tanks, filtration systems, etc.

The deposited object 20 comprise a matrix 22, and cells 24 at least partially embedded in the matrix. The matrix 22 is arranged in three-dimensional structure that forms cavities or pores 23. As the deposited object 20 is maintained in the bioreactor, the cells may grow to colonise the pores 23 within the deposited object 20.

**Figure 1B** shows a simplified process diagram for a generic bioprocess according to the disclosure. The illustrated process comprises optional step 120 of providing a bioink composition comprising a cell population and one or more biomaterials capable of forming a hydrogel. The bioink composition may be deposited into a three-dimensional structure by additive manufacturing at step 130, thereby forming a deposited object / cellularised structure. The bioink composition may then be consolidated at step 140. At step 250, the cellularised structure is cultured, resulting in the growth of the cell population. At step 160, one or more cellular products are harvested. This may include the step of removing some or all of the culture medium (optionally with replacement, for example where the harvesting step is performed during the course of cell culture rather than at the end of it) at step 160A. Instead or in addition to this, harvesting a cellular product may comprise separating the cells from the hydrogel matrix at step 160B, at the end of the culture. Embodiments of these generic steps are described in more detail by reference to Figures 2-4.

**Figure 2** illustrates schematically a process for production of cellular products according to embodiments of the present disclosure. At step 200, a population of cells (typically production cells, for example from a production cell line) is amplified using conventional cell culture protocols such as e.g. using a suspension culture or adherent culture. The choice of a suitable amplification protocol may depend on the cell type at hand. At step 210, the amplified cell population is harvested, for example by centrifugation and/or trypsinisation (depending on the cell culture protocol used at step 200. At step 220 a bioink is formulated by mixing the harvested cells with a solution comprising biomaterials capable of forming a hydrogel (in particular, gelatin, alginate and fibrinogen, in the illustrated embodiment). At step 230, the bioink composition is 3D printed, for example by pneumatic extrusion in the illustrated embodiment. At step 240, the bioink composition is consolidated. In the illustrated embodiment, the structure formed by the gelatin may at least partially dissolve and a more permanent structure is formed by cross linking the alginate and fibrinogen, respectively. The partial dissolution of the gelatin may create microporosities that advantageously enable further cell growth. At step 250, the cellularised structure is cultured in a culture medium, during which the cell population grows to colonise the structure. At step 260, the cells are harvested by dissociating the hydrogel matrix.

**Figure 3** illustrates schematically a process for providing a cellularised structure according to embodiments of the disclosure. At step 320, a solution of cells (such as e.g. cells suspended in culture medium or buffer, preferably calcium free culture medium such as DMEM(-)) is mixed with stock solutions respectively comprising 5 % w/v gelatin, 2 % w/v alginate and 2 % w/v fibrinogen. Preferably, each of these stock solutions are prepared in culture medium. Note that alternative concentrations of each of these components and compositions comprising two or all three of these biomaterials may be used instead. This solution may be referred to as a bioink. At step 325A, the bioink is incubated at 37C to allow the solution to homogenise and the cells to recuperate, for example for 10 minutes. At step 325B, the bioink is incubated at 21C to allow the composition to reach a viscosity suitable for printing, for example for 30 minutes. This may be performed in the same syringe that will be used for printing. Note that the precise times and temperatures provided are indicative only, and alternative times / temperatures may be used depending on the cell population, the composition of the bioink (ingredients and concentrations) and the desired printing viscosity (which can depend on the printing parameters). At step 330, the bioink is printed into a predetermined 3D structure. At step 340, the deposited object is consolidated by incubation with a consolidation solution. The timing of consolidation may depend on the concentration of the consolidation solution and the size of the object. Indicative times are provided. The temperature of consolidation may depend on the cells and on the composition of the bioink (for example, the temperature may be chosen to be sufficient to at least partially re-melt the gelatin without damaging the cells). At step 350, the consolidated cellularised structure is cultured in a bioreactor.

**Figure 4** illustrates schematically a process for harvesting of cells from cellularised structures by dissociation, according to embodiments of the disclosure. At step 460a, the celllularised structure is weighed to determine the parameters of the dissolution protocol. At step 460b, the cellularised structure is incubated in a solution of collagenase 3 % w/v in a PBS buffer, in the illustrated embodiment. Thus may be performed at 37C or any other temperature that is compatible with cell viability and action of the enzyme used for dissolution. The timing of incubation may depend on the object (e.g. size) and incubation may be maintained until complete dissociation of the object. This may take about 1 to 1.5 hours. At step 460c the solution is centrifuged to separate the cells from the supernatant comprising the dissolved matrix. At step 460d the cell pellet is resuspended, for example in PBS or culture medium. The volume of solution used may depend on the amount of cells and the subsequent steps to be performed. At step 460e, the cells are counted, for example using a cell counting device such as a hemocytometer or a flow cytometer. Any cell counting protocol known in the art may be used. It is then determined whether the cell solution comprises many cell aggregates. If that is not the case then the result of step 460e enables the determination of the final cell concentration as a result of the bioprocess. If too many cell aggregates are present (where the threshold for this may depend on the particular application), the cells may be briefly incubated with trypsin to break up these aggregates at step 460f. This may use trypsin 1x at 37C for 5 minutes or any other trypsinisation protocol known in the art. Note that this may only be performed if individualisation of the cells is necessary or desirable, but is not necessary for the harvesting of the cells per se. The trypsin may then be inactivated at step 460g, as trypsin may cause damage to the cells. This may involve the addition of culture media with foetal bovine serum. The resulting solution may then be centrifuged (step 460c), resuspended (step 460d) and re-counted (step 460e).

### Examples

Exemplary methods of producing a cellular product in a 3D printed hydrogel matrix, as well as exemplary methods for obtaining a cell culture in a 3D printed hydrogel matrix will now be described.

### Materials and Methods

Generally, cells were amplified in "traditional" culture in suspension or as adherent cultures in flask (depending on the cell type) (Fig. 2, step 200). The cells were then trypsinised (Fig. 2, Step 210), and formulated as a bioink formulation (Fig. 2, step 220). The formulation was 3D printed (Fig. 2 step 230), and the construct was consolidated (Fig. 2, step 240). After culture (Fig. 2, step 250), the cellularised structures were dissociated to harvest the cells (Fig. 2, step 260).

*Cell trypsinisation - HEK293 cell line.* HEK293 cells were cultivated in T75 flasks (Corning). The culture media was carefully discarded from the flasks and cells were gently rinsed with 5 mL PBS 1X (Gibco). PBS was removed from the flasks and 5mL of DMEM without calcium DMEM (-) (Gibco) was added in the flasks. Cells were detached from the flask by gently shaking them. The detached cells in DMEM (-) were recovered in a 50ml falcon tube and counted on an hematocymeter for cell concentration determination.

*Cell trypsinisation - MDCK cell line.* MDCK cells were prepared in T75 flasks (corning). The culture media was carefully discarded from the flasks and cells were gently rinsed with 5 mL PBS 1X (Gibco). PBS was removed and 10 mL of fresh PBS were added. The flasks were then incubated 40 min to 1h at 37°C. The PBS was then removed and 5 mL of trypsin-EDTA 1X (Trypsin : 0.25 g/L, 0.0105mM ; EDTA 4Na 2H2O : 0.038 g/L, 0.09130226 mM) (GibcoTM, Trypsin-EDTA (0.5%), no phenol red, 15400054) were added in each flask and incubated 2 to 5 min at 37°C to completely detach the cells. After tapping the flasks, 10 mL of DMEM(-) (Gibco) were added in each flask to inactivate the trypsin. The detached cells in DMEM(-) (Gibco) were recovered in a falcon 50mL tube and counted on an hematocymeter.

*Cell trypsinisation - CHO cell line.* CHO cells were cultivated in suspension in T75 flasks (Corning). The culture medium with the suspended cells was recovered in a 50ml falcon and centrifugated for 5 min at 300g. The bottom of the flask was rinsed with PBS 1X (Gibco) and 2 mL trypsin-EDTA 1X (Trypsin : 0.25 g/L, 0.0105mM ; EDTA 4Na 2H2O : 0.038 g/L, 0.09130226 mM) (GibcoTM, Trypsin-EDTA (0.5%), no phenol red, 15400054). were then added to detach the adhering cells. The flask was incubated 2 min at 37°C and then 5 mL of DMEM(-) (Gibco) was added to the flasks to inactivate trypsin. The detached cells were recovered and added to the tube containing the media with suspension cells. The cells were counted on an hematocymeter.

*Cell trypsinisation - VERO cell line.* VERO cells are cultivated in T75 flasks (Corning). The culture media was carefully discarded from the flasks and cells were gently rinsed with 5 mL PBS. PBS was removed prior addition of 2.5 mL of trypsin-EDTA 1X (Trypsin : 0.25 g/L, 0.0105mM ; EDTA 4Na 2H2O : 0.038 g/L, 0.09130226 mM) (GibcoTM, Trypsin-EDTA (0.5%), no phenol red, 15400054) were added in each flask and incubated 2 min at 37°C to completely detach the cells. After tapping the flasks, 5 mL of DMEM(-) were added in each flask to inactivate the trypsin. The detached cells in DMEM(-) were recovered in a falcon 50mL tube and counted on an hematocymeter.

*Stock solutions for bioink formulation.* Stock solutions were prepared the day prior bioprinting to allow for best component dissolution. Fibrinogen stock solution was prepared by dissolving 8% w/v Fibrinogen from bovine plasma (F8630-1G, Merck) in DMEM without calcium (Gibco). Alginate stock solution was prepared by dissolving 4% w/v Alginate (Alginic acid sodium salt very low viscosity, A18565.36, Alfa Aesar) in DMEM without calcium (Gibco). Gelatin stock solution was prepared was prepared by dissolving 20%w/v Gelatin from porcin skin (Sigma Aldrich, G1890) in DMEM without calcium (Gibco).

*Bioink formulation.* See Fig. 3, steps 320, 325A, 325B. After cell trypsinization (see above), targeted cell concentration is adjusted by pelleting the appropriate volume of cells at 300g during 5 min. The pellet was resuspended in the appropriate volume of Fibrinogen 8% solution. The appropriate volumes of Alginate 4% solution and the Gelatin 20% solution are further added with a final proportion 25% Fibrinogen solution, 25% Alginate solution and 50% Gelatin solution corresponding to a final concentration in the bioink of 2%w/v Fibrinogen, 2%w/v Alginate, and 5%w/v Gelatin (Fig. 3, step 320). This formulation ensures a rheofluidifying behavior of the bioink ensuring the protection of cells while printing. The cells and the three components of the bioink were mixed and homogenized by carefully pipetting 10 times with a viscous-liquid pipet. The bioink was then incubated 10 min at 37°C (Fig. 3, step 325A) before being poured into a printing sterile syringe. The bioink is then incubated 30 min at room temperature (21°C) before being used (Fig. 3, Step 325B) for cellularised structure 3D bioprinting.

*Bioink rheology.* The appropriate rheology of the bioink for printing of in particular large structures (e.g. above 10 cm$^3$) with porosity can be established by comparing a rheogram acquired for each composition with a standard (reference) rheogram for a bioink composition with known rheological properties (see **Figure 15A**). For a composition with these properties, a range of temperature compatible with printing, where the properties of the composition are such that high cell viability is maintained while allowing to maintain high fidelity of the deposited shape, was determined. This was determined by determining the range of values of maximum shear stress and static yield stress that are compatible with maintaining the shape fidelity of the bioprinted object, and the range of values of maximum shear stress and static yield stress that are compatible with maintaining the viability of the cells after microextrusion (assessed using cell viability of NIH fibroblast cells included in the bioink). The results of this for an exemplary bioink composition are shown on **Figure 15B,** where the shaded zone represents the shear stress (MSS) and static yield stress (SYS) that cells can handle (NIH viability limit) and that also provides suitable shape fidelity (STL fidelity). Based on the data on Figure 15B, a printing temperature between 21 and 28°C was chosen. The shear rate on the wall of the extrusion nozzle ($\dot{\gamma}_w$) was calculated in order to determine the shear stress experienced by the cells in the printing nozzle. To obtain the wall shear stress value, the shear rate in the syringe nozzle and the associated viscosity were independently determined. First, a stress controlled rotational rheometer (DHR2, TA instruments) was used to measure the static yield stress of bioink and the shear stress felt by cells during the bioprinting process. A plan-plan (40 mm) geometry was used with a shear rate sweep procedure at different temperatures (21, 23, 28 and 37°C). From this experiment, bioink shear stress was determined over a large shear rate scale [0.01; 100] s$^{-1}$. Second, the wall shear rate in the cartridge was defined from a traditional Poiseuille equation as a function of flow (equation 1):

$$\dot{\gamma}_w^{t,b} = \frac{3n+1}{n}\frac{Q}{\pi R_{t,b}^3} \qquad (1)$$

where n is the flow behavior index determined in the rotational rheological experiment, Q (mm$^3$.s$^{-1}$) is the flow and $R_{t,b}$ (mm) is the radius at the top (t) and bottom (b) of syringe nozzle. A flow value of 0.183 mm$^3$.s$^{-1}$was used in these experiments. As the printing nozzle used was frustoconical, the wall shear stress increases from the top to the bottom of frustoconical printing nozzle and results were therefore obtained for both the top and bottom of the nozzle.

*Formulation and culture - HEK293 cell line.* Experiments were carried out with HEK 293T cells seeded at different cell densities, these cell densities were ranging from $1 \times 10^6$ cells/ mL to $10 \times 10^6$ cells/ mL of bioink. Cellularised square structures of 0.2cm$^3$ or tubes were cultivated in 2mL culture medium.

*Formulation and culture - MDCK cell line.* Experiments were carried out with MDCK cells seeded at a density of $1 \times 10^6$ cells/ mL of bioink and $3 \times 10^6$ cells/ mL of bioink. Tissues of 0.2cm$^3$ were cultivated in 2mL culture media corresponding respectively to cell concentrations of $1 \times 10^5$ cells/ mL of medium and $3 \times 10^5$ cells/ mL of medium.

*Formulation and culture - CHO cell line.* Experiments were carried out with CHO cells seeded at a density of $1 \times 10^6$ cells/ mL of bioink. Tissues of 0.2cm$^3$ were cultivated in 2mL culture media corresponding to a cell concentration of $1 \times 10^5$ cells/ mL of medium.

*Formulation and culture - VERO cell line.* Experiments were carried out with VERO cells seeded at a density of $3 \times 10^6$ cells/ mL of bioink. Tissues of 0.2cm$^3$ were cultivated in 2mL culture media corresponding to a cell concentration of $3 \times 10^5$ cells/mL of medium.

*3D printing.* SeeFig. 3, step 330. The printed structures were designed by Computer Assisted Design (CAD) using 3D modelling software like Autodesk® Fusion 360™ and 3D Builder (Microsoft Corporation). 3D CAD files were converted to .stl format to be transferred to 3D printer equipment. The cellularized structure were bioprinted by pneumatic microextrusion using a BioAssemblyBot 3D printer (Advanced Solutions, Inc). The bioink was prepared as described above and placed within printing syringes of 10cc or 30cc according to the volume of bioink prepared. The needles used with these syringes had diameters ranging from 200 μm to 800 μm diameter. The printing process was carried out at 21°C with pneumatic pressures ranging from 20 to 50 psi. These ranges were found to result in the formation of a a smooth continuous filament.

*Cellularised structure consolidation.* See Fig. 3,step 340. The bioprinted tissues were consolidated with a solution of CaCl$_2$ (C5670-500G, Merck), transglutaminase (TAG) (ACTIVA WM, Ajinomoto) and thrombin (Thrombin from bovin plasma, t4648-10KU, Merck) in deionized water. Preferred concentrations are 3% w/v CaCl$_2$, 4% transglutaminase and 10 U/mL of Thrombin. The consolidation process was carried out at 37°C for 1 hour for the small tissues of 0.2 cm$^3$ and for 2 hours for the large tissues above 10 cm$^3$. The cellularized structures were then rinsed twice with sterile physiological serum at a ratio of 10 mL physiological serum for 1mL of tissue.

*Culture and harvesting* of *secreted molecules.* After consolidation of the cellularized structures, these were cultured in non-treated 12-well plates with 2mL of their specific culture medium (see Fig.3, step 350). Culture media were renewed 3 times a week. Before each media exchange, the plates were observed under the microscope to evaluate cell dispersion on the well bottom. If cells that were no longer part of the cellularised structures were observed on the well bottoms, the cellularised structures were transferred to new 12-well plates. This was to ensure that the observations related exclusively to cells embedded within the structure. In use the presence of additional cells either in the culture medium or on any surface in the culture vessel may not be problematic and may be tolerated. Spent media were recovered in triplicate for each condition and were stored at -20°C. These samples were used to quantify the molecules secreted. The HEK293 tissues were cultured in DMEM Glutamax supplemented with 10% FBS (fetal bovine serum). The MDCK tissues were cultured in DMEM Glutamax supplemented with 10% FBS. The CHO tissue cultivation experiments were caried out using DMEM Glutamax supplemented with 10% FBS and 4Cell® XtraCHO Media (Stock & Adaptation, Production, Feed Medium A, Feed Medium B).

The VERO tissue cultivation experiments were caried out using VP-SFM (1X) (11681020, Gibco).

*Cell harvesting by dissociation - dissociation with collagenase* A 3% *In PBS (phosphate-buffer saline).* A collagenase A **(COLLA-RO** Roche) 3% w/v (4.5 U/mL) was prepared in PBS 1X (Gibco) solution and warmed at 37°C in a water bath. The tissues were rinsed once with 2mL PBS 1X (Gibco), then weighed (Fig. 4, step 460a) before being immersed in the collagenase solution (0.5 mL for a 0.2 cm$^3$ tissue) (Fig. 4, step 460b). Cellularised structures were then incubated at 37°C in a water bath with vigorous agitation every 10 to 20 minutes until complete dissociation (-40 min - 1h for a 0.2 cm$^3$ tissue). The tubes were centrifugated (Fig. 4, step 460c) and the cell pellets were resuspended in 1mL PBS (Fig. 4, step 460d). Cells were counted on an hematocymer with 0.4 % trypan blue staining (Fig. 4, step 460e).

*Cell harvesting by dissociation - dissociation with collagenase* A 3% *in DMEM without calcium.* A collagenase A **(COLLA-RO** Roche) 3% w/v (4.5 U/mL) was prepared in DMEM without calcium (Gibco). The tissue dissociation process was then caried out as described above.

*Cell harvesting by dissociation - collagenase dispase.* A solution of collagenase/dispaseR (COLLDISP-RO Roche) 1mg/mL (Collagenase: 0.1U/mL, Dispase: 0.8U/mL) in PBS was heated at 37°C in a waterbath. The cellularised structures were rinsed once with PBS 1X (Gibco), then weightd before being immersed in the collagenase/dispase solution (1 mL for a 0.2 cm3 tissue) and incubated at 37°C in a waterbath with vigorous agitation every 10 to 20 minutes, during 2h. The tubes were then incubated overnight at 4°C. The tubes were centrifugated and the cell pellets were resuspended in 1mL PBS. Cells were counted on an hematocymer with 0.4 % trypan blue staining

*Cell harvesting by dissociation - trypsin-EDTA. A solution* of trypsin-EDTA 1X (Trypsin: 0.25 g/L, 0.0105mM ; EDTA 4Na 2H2O : 0.038 g/L, 0.09130226 mM) (GibcoTM, Trypsin-EDTA (0.5%), no phenol red, 15400054) was heated at 37°C in a water bath. The cellularised structures were rinsed once with 2mL PBS 1X (Gibco), then weighed before being immersed in the trypsin solution (1 mL for a 0.2 cm$^3$ cellularised structure) and incubated at 37°C in a waterbath with vigorous agitation every 10 to 20 minutes.

*Cell harvesting by dissociation - TrypLE.* TrypLE™ Express 1X solution (TrypLE™ Express Enzyme (1X), no phenol red, 12604013, GibcoTM) was heated at 37°C in a waterbath. The cellularised structures were rinsed once with PBS 1X (Gibco), then weighed before being immersed in the TrypLE solution (1 mL for a 0.2 cm$^3$ cellularised structure) and incubated at 37°C in a waterbath with vigorous agitation every 10 to 20 minutes.

*Cell harvesting by dissociation - Pepsin.* A Pepsin solution (Pepsin/HCl cleaning solution, Mettler Toledo, 51350100) was used at pH 1.82 and with a pH adjusted to 8.5 with NaOH. The cellularised structures were rinsed once with PBS 1X (Gibco), then weighed before being immersed in the Pepsin solution (1 mL for a 0.2 cm$^3$ cellularised structure) and incubated at 37°C in a waterbath with vigorous agitation every 10 to 20 minutes.

*Cell harvesting by dissociation - GentleMACS.* The cellularised structures were rinsed once with PBS 1X (Gibco), then weighed before being immersed in 5 mL PBS in a gentleMACS™ Dissociator tube. The tube was placed in the gentleMACS™ Dissociator (Miltenyi Biotec) and the program lung_02.1 was used 3 times simultaneously. The cells mixture was recovered from the spinning tube, the tube was rinsed twice with 5 mL PBS. The PBS used for rinsing the spinning tube was recovered in the same tube as the cell mixture. The tubes were centrifugated and the cell pellets were resuspended in 1mL PBS. Cells were counted on an hematocymer with 0.4 % trypan blue staining.

*Cell harvesting by dissociation - EDTA.* A solution of EDTA (EthyleneDiamine Tetraacetic Acid, tetrasodium - dihydrate- MW : 416.2 g/mol - CALBIOCHEM - 34103) at 50 mM was prepared by dissolving 0.4162g in 10 mL PBS 1X (Gibco), pH was then adjusted to 7 with HCl (pH 7.15) and the volume was completed to 20 mL with PBS. The solution was heated at 37°C in a waterbath. The cellularised structures were rinsed once with PBS 1X (Gibco), then weighed before being immersed in the EDTA solution (0.5 mL for a 0.2 cm$^3$ cellularised structure) and incubated at 37°C in a waterbath with vigorous agitation every 10 to 20 minutes.

*Cell harvesting by dissociation - Sodium Citrate.* A Sodium citrate (Sodium citrate monobasic anhydrous - MW: 214.11 g/mol - Sigma - 71497) 55 mM solution was prepared by dissolving 0.235g g in 10 mL PBS 1X (Gibco), pH was then adjusted to 7 with NaOH (pH 7.3) and the volume was completed to 20 mL with PBS. The solution was heated at 37°C in a waterbath. A Sodium citrate 100 mM solution was prepared by dissolving 0.428g in 10 mL PBS 1X (Gibco), pH was then adjusted to 7 with NaOH (pH 6,9) and the volume was completed to 20 mL with PBS. The solution was heated at 37°C in a waterbath. The tissues were rinsed once with PBS, then weighed before being immersed in the sodium citrate solutions (1 mL for a 0.2 cm$^3$ tissue) and incubated at 37°C in a waterbath with vigorous agitation every 10 to 20 minutes.

*Cell harvesting by dissociation - Alginate lyase followed by collagenase* A. A solution of Alginate lyase 5U/mL was prepared by dissolving 0.5 mg/mL of Alginate lyase (A1603-100MG, Sigma Aldrich) in PBS 1X (Gibco). A solution of collagenase A (COLLA-RO Roche) 3% w/v in PBS 1X (Gibco) (4.5 U/mL) was prepared. Both solutions were heated at 37°C in a waterbath. The cellularised structures were rinsed once with PBS 1X (Gibco), then weighed before being immersed in the Alginate lyase solution (0.5 mL for a 0.2 cm$^3$ cellularised structure) and incubated 15 min at 37°C in a waterbath. After 15 min, the cellularised structures were still in one piece but seemed to be fragilized. The tubes were then centrifugated to remove de Alginate lyase solution and the tissue was rinsed with 1 mL PBS before being centrifugated again to remove the PBS. The cellularised structure was then immersed in the collagenase solution (0.5 mL for a 0.2 cm$^3$ cellularised structure) and incubated at 37°C in a waterbath until complete dissociation. The tubes were centrifugated and the cell pellets were resuspended in 1mL PBS. Cells were counted on an hematocymeter with 0.4 % trypan blue staining. Note that a similar protocol could be used in which the cellularised structures are first incubated in collagenase then in alginate lyase. However, the incubation in alginate lyase then collagenase is advantageous in this case because the amount of gelatin is higher than the amount of alginate. Therefore, the short incubation in alginate lyase is enough to dissolve the alginate, and then the object can be left in the collagenase until the structure is completely dissolved. The assessment of whether the incubation in collagenase was sufficient may be more difficult to make if the incubation in collagenase precedes the incubation in alginate lyase.

*Cell harvesting by dissociation - Alginate lyase-collagenase* A. A solution of collagenase A (COLLA-RO Roche) 3% w/v (4.5 U/mL) in PBS 1X (Gibco) was prepared. A collagenase A / Alginate lyase solution was prepared by dissolving 0.5mg/mL of Alginate lyase (A1603-100MG, Sigma Aldrich) in 3% collagenase solution (Alginate lyase 5 U/mL; Collagenase 4.5 U/mL). The collagenase A / Alginate lyase was heated at 37°C in a waterbath. The cellularised structures were rinsed once with PBS 1X (Gibco), then weighed before being immersed in the collagenase/alginate lyase solution (0.5 mL for a 0.2 cm$^3$ cellularised structure) and incubated at 37°C in a waterbath with vigorous agitation every 10 to 20 minutes, until complete dissociation. The tubes were centrifugated and the cell pellets were resuspended in 1mL PBS. Cells were counted on an hematocymeter with 0.4 % trypan blue staining.

*Cell harvesting by dissociation - sodium citrate followed by Alginate lyase-collagenase* A. The cellularised structures were rinsed once with PBS 1X (Gibco), then weighed before being immersed in 1mL of 100mM sodium citrate solution (section 6.9) pre-heated at 37°C, and incubated 15 minutes at 37°C in a waterbath. The tubes were then centrifugated to remove de sodium citrate solution and the cellularised structure was rinsed with 1 mL PBS before being centrifugated again to remove the PBS. The cellularised structure was then immersed in 0.5 mL of a Alginate lyase / Collagenase A solution (see *"Cell harvesting by dissociation - Alginate lyase-collagenase A"*) and incubated at 37°C in a waterbath until complete dissociation (-35 min) The tubes were centrifugated and the cell pellets were resuspended in 1mL PBS. Cells were counted on an hematocymeter with 0.4 % trypan blue staining.

*Cell harvesting by dissociation - Human platelet lysate (HPL).* HPL was pre-heated at 37°C in a waterbath. The cellularised structures were rinsed once with PBS 1X (Gibco), then weighed before being immersed in 1.5 mL of HPL and incubated at 37°C in a waterbath until complete dissociation. The tubes were centrifugated and the cell pellets were resuspended in 1mL PBS. Cells were counted on an hematocymeter with 0.4 % trypan blue staining.

*Cell harvesting by dissociation - Alginate Lyase + EDTA + sodium citrate.* A solution was prepared by mixing 0.5 mL of Alginate lyase (5 U/mL) solution (section 6.8), 0,5 mL EDTA (see *"Cell harvesting by dissociation - EDTA")* and 0.5 mL sodium citrate 100 mM (section 6.9), and pre-heated at 37°C. The cellularised structures were rinsed once with PBS, then weighed before being immersed in 1.5 mL of this solution and incubated at 37°C in a waterbath.

*Cell harvesting by dissociation - EDTA + sodium citrate.* A solution was prepared by mixing 2 mL EDTA (section 6.10) and 2 mL of a sodium citrate 100 mM solution (see *"Cell harvesting by dissociation - Sodium Citrate"*), and pre-heated at 37°C. The cellularised structures were rinsed once with PBS, then weighed before being immersed in 1.5 mL of this solution and incubated at 37°C in a waterbath.

*Monitoring of live and dead cells with fluorescent labels.* The culture medium was removed, and the cellularised structures were rinsed with a volume of PBS 1X (Gibco) equivalent to the volume of culture medium used in the culture well. PBS was then discarded and replaced by a volume of 2μM calcein green (Calcein, AM, cell-permeant dye, C1430, Invitrogen™) allowing complete immersion of the celularised structure (1mL for a 0.2 cm$^3$ tissue). The cellularized structures were then incubated in calcein green solution at 37°C for 30 min. The cellularised structures were then rinsed with PBS and immersed in 300 nM DAPI (4',6-Diamidino-2-Phenylindole, Dihydrochloride) (Invitrogen™, D1306) (1 mL for a

0.2 cm$^3$ cellularised structure) and incubated 10 min at 37 °C in the dark in an incubator. Live (green calcein staining) and dead (light blue DAPI staining) cells were observed in a dark room with a fluorescence microscope (Nikon eclipse Ts2R) equipped with 490 nm and 340 nm filters. Live cells were visible as green, fluorescent cells and nucleus of dead cells were stained in light blue.

*Quantification of secreted lactic acid.* Culture spent medium was collected in the 12-well plates before each media exchange. The lactic acid concentration was quantified using the L-Lactic Acid Assay from Megazyme (L-Lactic Acid (L-Lactate) Assay Kit, K-LATE, Megazyme) for auto-analyzer procedures. The assay was performed in 96-well plates. 200 μL of reactive R1 was deposited in the wells followed by addition of 10 μL of the samples and 25 μL of reactive R2. Each sample was deposited twice (duplicata) in the 96 well plate. The plates were incubated at 25°C in the dark for 10 min before measurement of the absorbance at 340 nm with a spectrophotometer (TECAN infinite®). If the samples were too concentrated, they were diluted in deionized water. Fresh culture medium was used as a negative control for the supernatant samples.

*Growth monitoring by spheroid measurement.* For the cells growing in spheroids, microscopic pictures of bioprinted structures were taken. Using microscope scale bars, pictures were analyzed using the software ImageJ to measure spheroids diameters of at least 6 to 10 spheroids (due to a high variability in spheroids diameters).

*Transduction in cellularised structures.* Cellularized structure were infected at various growth phases with viral vectors expressing fluorescent green protein GFP (either GFP-lentivirus or GFP-Baculovirus BacMam). Infection process was performed with media supplemented with concentrated viral vectors of various concentrations. GFP expression was observed from 2 days post-infection up to 6 days post-infection.

### *Example 1 - Culture of production cell lines in 3D printed structures*

In this example, a plurality of production cell lines were cultured embedded in 3D printed hydrogel structures, in order to investigate the production potential of the approach.

The results for CHO cells are shown on **Figure 5.** Cells were seeded at $1 \times 10^6$ cell/mL of bioink (eq. to 1.0 $\times 10^5$ cell/ mL of medium) within a culture medium composed of DMEM and FBS (10%). The data shows that cell concentrations of about $2\times10^5$ cells/ml are reached after 20 days of culture (square data points), without any optimization, and that the density of live cells increases during culture, illustrating strong potential for growth, and long term viability in culture.

The results for HEK cells are shown on **Figure 6.** Cells were seeded at $1 \times 10^6$cell/mL of bioink (eq. to 1.0 $\times 10^5$ cell/ mL of medium) within a culture medium composed of DMEM and FBS (10%). The data shows that cell concentrations of about $6\times10^5$ cells/ml are reached after 20 days of culture (square data points), without any optimization, and that the density of live cells increases during culture, illustrating strong potential for growth, and long term viability in culture.

The results for MDCK cells are shown on **Figure 7.** Cells were seeded at $1 \times 10^6$ cell/mL of bioink (eq. to 1.0 $\times 10^5$ cell/ mL of medium) within a culture medium composed of DMEM and FBS (10%). The data shows that cell concentrations of about $3\times10^5$ cells/ml are reached after 20 days of culture (square data points), without any optimization, and that the density of live cells increases during culture, illustrating strong potential for growth, and long term viability in culture.

The results for VERO cells are shown on **Figure 8.** Cells were seeded at $3 \times 10^6$ cell/mL of bioink within a VP-SFM (1X) culture medium (11681020, Gibco). The data shows that cell concentrations of about $3\times10^5$ cells/ml are reached after 20 days of culture (square data points), without any optimization, and that the density of live cells increases during culture, illustrating strong potential for growth, and long term viability in culture.

### *Example 2 - Growth optimisation through seeding density*

In this example, the effect of the seeding density on the growth obtained in 3D printed cellularised structures was investigated. The results of this are shown on **Figure 9.** HEK293 and MDCK cells were seeded at different densities then printed as explained above and cultured for 24 days (576 hours). The culture medium was changed every 2 days. The data shows that similar final cell densities can be obtained for MDCK cells within a range of seeding densities, whereas HEK293 cells seem to reach higher final cell densities in cellularised structures if they are initially seeded at lower densities. The data therefore shows that optimisation of cell seeding density may lead to further improvements in cell production capability with the approach described herein at least for some cell lines.

### *Example 3 - Investigation of dissociation protocols*

In this example, a plurality of dissociation protocols were evaluated for their suitability for cell harvesting from cellularised structures, in cell production (i.e. gel dissociation and (viable) cell recovery). The results of this analysis are shown in **Table 1** below.

**Table 1.** Evaluation of cell viability, total cell recovery and hydrogel dissociation efficiency for a plurality of dissociation protocols.

| Protocol | Cell viability (%) | Cell total recovery (%) | Hydrogel dissociation |
|---|---|---|---|
| 3% Collagenase A in PBS *** | 22 % | 100% | Efficient dissociation of the tissue after 40 min to 2h, final solution is not very "clean", presence of micro-debris |
| 3% Collagenase A in DMEM medium (without FBS) *** | 25% | 100% | Efficient dissociation of the tissue after 40 min to 2h, final solution is not very "clean", presence of micro-debris |
| Collagenase/Dispase *** *1mg/mL (Collagenase: 0.1U/mL, Dispase: 0.8U/mL)* | 16% | 100% | Efficient dissociation of the tissue after 2h at 37°C and overnight incubation at 4°C. final solution is not very "clean", presence of micro-debris |
| Trypsin 1X *** | NA | 0 | No dissociation |
| TrypLE 1X *** | NA | 0 | No dissociation |
| Pepsin 0.5% *** | NA | 0 | No dissociation |
| Mechanical dissociation **** *3 times protocol GentleMACS lungs_01* | NA | <5% | Dissociation of the tissue with high cell mortality and presence of numerous large debris |
| EDTA * | NA | 0 | No dissociation |
| Sodium citrate * *55mM and 100 mM* | NA | 0 | Fragmentation of the gel in large pieces but dissociation incomplete |
| Alginate lyase followed by Collagenase A ** | 44.4 | 73.8 | Efficient dissociation of the tissue after 1h15. Solution is clean with presence of rare micro-debris |
| Alginate lyase / Collagenase A ** | 33.8 | 29.6 | Efficient dissociation of the tissue after 1h15, presence of few micro-debris |
| $Na^{2+}$-citrate followed by Alginate lyase/CollA ** | 35.1 | 84.7 | Efficient dissociation of the tissue after 1h15. Hydrogel is perfectly dissociated and solution is very clean with no debris |
| Human Platelet Lysate * | NA | NA | Dissociation duration and final fragment size equivalent to collagenase dissociation |
| Alginate lyase + EDTA + sodium citrate * | NA | 0 | Fragmentation of the gel in large pieces but dissociation incomplete |
| EDTA + sodium citrate * | NA | 0 | Fragmentation of the gel in large pieces but dissociation incomplete |
| Experiments (*) were carried out with non-cellularized molded tissues to evaluate the efficiency of hydrogel dissociation - this is why there is no data for cell recovery in these experiments. Experiments (**) were carried out with molded tissues cellularized with $2 \times 10^6$ HEK 293T/ml of bioink. Experiments (***) were carried out with bioprinted tissues cellularized with $2.6 \times 10^6$ HEK 293T/ml of bioink. GentleMACS (****) experiment was carried out with bioprinted tissues cellularized with $1.5 \times 10^6$ HEK 293T/ml of bioink. | | | |

The data shows that protocols that are normally used for tissue dissociation, such as using collagenase, collagenase and dispase or alginate lyase are particularly advantageous in the context of the approaches described as they enable high cell recovery with high viability. Amongst these, the use of collagenase appears to be particularly advantageous as it leads to high cell recovery and viability. Further, the use of collagenase in combination with alginate lyase (whether concomitantly or in succession) appears to be particular advantageous as it reduces the presence of micro-debris.

### Example 4 - Recombinant protein production

In this example, inventors demonstrate the potential for the methods described herein to be used for the production of recombinant proteins. The results of this are shown on **Figures 10** (A. fibroblasts, B. VERO and C. MDCK cells infected with GFP-viruses) **and 11** (HEK293 cells 3D printed at cell density of $3 \times 10^6$ cell/ml bioink and infected with GFP-lentivirus). The data shows that successful growth of the cells and infection in the cellularised structure was obtained, followed by successful expression of the recombinant protein (in all cell lines tested).

### Example 5 - Metabolite production

In this example, inventors demonstrate the potential for the methods described herein to be used for the production of metabolites. The results of this are shown on **Figure 12.**

Lactic acid secreted by HEK293 cells in a cellularised structure was quantified in the culture medium collected from the process at every medium exchange, without the need for prior separation (filtration/ centrifugation). The data shows that the metabolite was efficiently secreted throughout the culture and could be efficiently harvested without disrupting the culture in any way.

### Example 6 - Optimisation of consolidation and printing parameters

In this example, the effect of various consolidation and printing parameters on production of a metabolite (in this case, lactate secretion, which is also an indicator of growth) in a cellularised structure was evaluated using a design of experiments (DOE) approach.

In particular, HEK293 cells were printed at $3 \times 10^6$ cell/mL of bioink, using different filament sizes and generating different porosities of the 3D object during printing. In particular, porosities of 0% (solid object) and 50% (cubic lattice comprising channels of square section) were tested (see **Figure 14**). The bioprinted constructs were then consolidated using different temperature, duration and concentrations of the polymerization solutions (see Table 2). The tissues were then kept in culture up to 6 days of culture. Culture media were sampled for lactic acid analysis (growth marker for these assays) and exchanged every 2 to 3 days. Calcein staining was used to evaluate cell viability (Calcein staining allows to identify live cells with microscopic observations). The degradation and resistance of the biomaterial was monitored along the cultivation process by evaluating the % of remaining slabs in culture.

**Table 2.** Description of the fractional factorial design of experiment implemented to evaluate printing and consolidation parameters. TAG=transglutaminase.

| | Consolidation | | | | | Printing | |
|---|---|---|---|---|---|---|---|
| *Condition* | *Temperature (°C)* | *Duration (min)* | *[TAG] (%w/v)* | *Thrombin* | *[CaCl$_2$] (%w/v)* | *Porosity* | *Filament diameter (μm)* |
| *1* | *21* | *60* | *0.04* | *No* | *3* | *Yes* | *400* |
| *2* | *37* | *15* | *0.04* | *No* | *0.03* | *No* | *400* |
| *3* | *21* | *60* | *4* | *No* | *0.03* | *No* | *200* |
| *4* | *37* | *60* | *0.04* | *Yes* | *0.03* | *Yes* | *200* |
| *5* | *37* | *15* | *4* | *No* | *3* | *Yes* | *200* |
| *6* | *37* | *60* | *4* | *Yes* | *3* | *No* | *400* |
| *7* | *21* | *15* | *0.04* | *Yes* | *3* | *No* | *200* |
| *8* | *21* | *15* | *4* | *Yes* | *0.03* | *Yes* | *400* |

The results of this are shown on **Figure 13.** The data shows that the parameters impacting the most the lactic acid secretion were the geometry (porous geometry is preferred with small diameter of the filaments -200μm) and the consolidation temperature (TA : 21°C is preferred).

Further, the impact of porosity, size and volume ratio between printed object and culture conditions (volume of culture medium) was evaluated by combining and comparing the data obtained for various combinations of these combinations of parameters with different cell lines (see Figure 14 for an illustration of the geometries used). The results of this are show in Table 3.

**Table 3.** Summary of results evaluating different porosities, size of objects and ratios of volume of printed object and culture medium. A/L=air/liquid interface for gaz diffusion (Y=yes, N=no air liquid interface); P=porosity; VO= Total volume of the object; VB= Volume of bioink in the object; VV=Volume of the cultivation vessel; VM=volume of culture medium; R= Ratio between Volume of media and volume of bioink; SCB= Seeded cells / $cm^3$ of bioink; SCM= Seeded Cells / $cm^3$ of cultivation media

| A/L | P (%) | VO ($cm^3$) | VB ($cm^3$) | VV ($cm^3$) | VM (mL = $cm^3$) | R (VM / VB) | SCB | SCM | Maximal cells density reached / $cm^3$ of bioink |
|---|---|---|---|---|---|---|---|---|---|
| Y | 0 | 0.2 | 0.2 | 3.3 | 2 | 10 | 3.0E+06 | 3.00E+05 | Not determined |
| | 50 | 0.2 | 0.1 | 3.3 | 1 | 10 | 3.0E+06 | 3.00E+05 | Not determined |
| | 0 | 0.2 | 0.2 | 6.6 | 2 | 10 | 3.0E+06 | 3.00E+05 | HEK293 : 1.95E+06 MDCK : 3.37E+06 VERO : 2.27E+06 |
| | 0 | 0.2 | 0.2 | 6.6 | 2 | 10 | 1.0E+06 | 1.00E+05 | HEK293 : 6.34E+06 MDCK : 2.25E+06 CHO : 3.02E+06 |
| | 50 | 0.4 | 0.2 | 6.6 | 2 | 10 | 3.0E+06 | 3.00E+05 | Not determined |
| N | 50 | 10.8 | 5 | 14 | 100 | 20 | 1.0E+06 | 5.00E+04 | Fibroblast : 1.01E+05 |
| | 50 | 10.8 | 5 | 14 | 200 | 40 | 3.0E+06 | 7.50E+04 | HEK293 : 2.21E+06 |

Thus, the data on Figure 13 shows that optimisation of printing geometry and structure of the hydrogel (in particular through optimising the cross-linking process during consolidation, such as e.g. by optimising the consolidation temperature) can be easily influenced to further improve the production of a secreted metabolite in any production set up. The data further shows that when using this sort of sizes of printed objects, a porous structure is advantageous for optimising growth. All of these parameters can be easily changed and optimised for any cell line and production set up, without requiring a material change such as the production of a new bioreactor or carrier, which is a major advantage of the present invention.

The data in Table 3 shows that good cell densities can be obtained for many cell lines in both porous and non-porous objects (particularly when the objects are small, such as below 1 $cm^3$), as well as for a variety of cell seeding densities and ratios of volumes of bioink and culture medium. In particular, ratios of volume of culture medium to volume of bioink between 5 and 50 (such as e.g. between 10 and 40 as demonstrated in the experiments in Table 3) are believed to be particularly advantageous. Indeed, previous experiments with a total volume of bioink printed of 5 $cm^3$ and 10 $cm^3$ within a cultivation vessel of 500ml (leading to a Ratio between Volume of media and volume of bioink of between 50 and 100) did not result in the high growth observed in Table 3 (data not shown).

*Equivalents and Scope*

All documents mentioned in this specification are incorporated herein by reference in their entirety.

Unless context dictates otherwise, the descriptions and definitions of the features set out above are not limited to any particular aspect or embodiment of the invention and apply equally to all aspects and embodiments which are described.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about" or "approximately", it will be understood that the particular value forms another embodiment. The terms "about" or "approximately" in relation to a numerical value is optional and means for example +/- 10%.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

Other aspects and embodiments of the invention provide the aspects and embodiments described above with the term "comprising" replaced by the term "consisting of" or "consisting essentially of", unless the context dictates otherwise.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

## Claims

1. A method for performing a bioproduction process comprising the production of a cellular product by a cell population, the method including the step of culturing the cell population at least partially embedded in a hydrogel matrix forming a three-dimensional structure.

2. The method of claim 1, wherein the hydrogel matrix has been obtained by providing a bioink composition comprising one or more biomaterials capable of forming a hydrogel and a cell population in a controlled three-dimensional shape to obtain a cellularised structure, optionally wherein the bioink composition comprises a liquid culture medium and/or wherein the bioink composition has been obtained by mixing one or more solutions each comprising biomaterials capable of forming a hydrogel and/or cells in a liquid culture medium.

3. The method of claim 1 or claim 2, wherein the cell population comprises or consists of cells from one or more cell lines, wherein the cell population does not comprise primary cells, wherein the cell population comprises or consists of cells from a single cell line, wherein the cell population grows as single cells or clusters of cells such as spheroids, wherein the cell population does not form a tissue in or on the hydrogel structure, and/or wherein the cell population comprises or consists of cells from one or more production cell lines, optionally wherein the one or more production cell lines are selected from: an MDCK cell line, AGE.CR1™, PRE.C6, a VERO cell line, EB.14, EP.66™, HEK293, BHK21, a CHO cell line, NS0, Sp2, Sf9, SF21, MRC-5, WI-38, a CEF cell line, and a hybridoma cell line, optionally wherein the one or more production cell lines are selected from a VERO cell line, a CHO cell line, an MDCK cell line, and HEK293.

4. The method of any preceding claim, wherein the three-dimensional structure has one or more internal cavities and/or wherein the three-dimensional structure forms a porous structure, optionally wherein the one or more internal cavities or pores have a controlled size distribution and/or wherein the one or more internal cavities or pores have a size within a predetermined range of sizes and/or wherein the three-dimensional structure comprises a plurality of internal cavities arranged on a regular lattice.

5. The method of any preceding claim, wherein the three-dimensional structure has been obtained by additive manufacturing, optionally wherein the method comprises obtaining a three-dimensional structure comprising a hydrogel matrix by additive manufacturing; and/or wherein obtaining a three-dimensional structure by additive manufacturing comprises depositing a composition at a rate below 0.2 mm$^3$/s; and/or
wherein obtaining a three-dimensional structure by additive manufacturing comprises depositing a composition as a filament, optionally wherein the filament has a diameter between 200 and 800 $\mu$m.

6. The method of any preceding claim, wherein the method comprises providing a bioink composition, depositing the bioink composition in a controlled three-dimensional shape to obtain a cellularised structure and/or consolidating the deposited bioink composition, preferably wherein consolidating the bioink composition comprises exposing the deposited bioink composition to one or more solutions that cross-link one or more of the biomaterials capable of forming a hydrogel and/or wherein providing the bioink composition comprises incubating the bioink composition at a predetermined temperature for a predetermined period of time such that the rheological properties of the bioink composition are compatible with printing without loss of cell viability.

7. The method of any preceding claim, wherein the hydrogel matrix comprises alginate and fibrin, and/or wherein the hydrogel matrix has been obtained by consolidation of a composition comprising alginate, fibrinogen and gelatin, optionally wherein the composition comprises between 1.75% and 26% w/v of gelatin, between 0.15% and 4.2% w/v of alginate, and between 0.15% and 5.25% w/v of fibrinogen and/or wherein consolidation of the composition comprises exposing the composition to a calcium salt and thrombin and/or wherein the composition is deposited in conditions enabling the gelatin in the composition to solidify thereby at least temporarily maintaining the 3D structure of the deposited composition.

8. The method of any preceding claim, wherein the cell population and hydrogel matrix structure together form a cellularised structure and culturing the cell population at least partially embedded in a hydrogel matrix forming a three-dimensional structure comprises maintaining the cellularised structure in a liquid culture medium in a bioreactor, preferably wherein the cellularised structure is completely immersed in the culture medium, optionally wherein the culture medium is a chemically defined culture medium, wherein the culture medium is a serum free culture medium, wherein the culture medium is a protein-free culture medium, and/or wherein the culture medium is free of additives used to protect the cells from hydrodynamic damage, optionally wherein the culture medium is free of detergents.

9. The method of any preceding claim, wherein the method comprises maintaining the cell population until it reaches a predetermined cell density, optionally wherein the predetermined cell density is at least 10$^8$ cell/ml, at least 10$^9$ or at least 10$^{10}$ cell/ml, and/or wherein the method comprises culturing the cell population for at least 7 days, at least 10 days, at least 15 days, or at least 20 days; and/or wherein the method comprises harvesting the cellular product, optionally wherein the step of harvesting the cellular product is performed one or more times during the culturing step and/or at the end of the culturing step; and/or wherein the method further comprises providing a cellularised structure comprising the cell population and hydrogel matrix prior to the culturing, wherein the cell density in the cellularised structure is between 1.10$^5$ and 1.10$^7$ cell / ml of hydrogel matrix or of the composition from which the hydrogel matrix is formed.

10. The method of claim 8 or claim 9, wherein the method comprises exchanging at least part of the culture medium at least once during the bioproduction process, and/or wherein the method comprises providing a flow of culture medium in the bioreactor, and/or wherein the method comprises a step of washing the cellularised structure, and/or wherein the method comprises maintaining the cellularised structure in a bioreactor comprising one or more inlets and one or more outlets to enable the addition of one or more solutions and the removal of culture medium from the bioreactor, optionally wherein the bioreactor comprises a flow control device to control the flow of solution/medium in and out of the bioreactor.

11. The method of any of claims 8 to 10, wherein the cellular product comprises a biological compound or structure secreted or otherwise released by the cells in the cell culture medium, optionally wherein the method comprises harvesting the cellular product by at least partially removing the culture medium, preferably wherein harvesting the cellular product does not comprise a cell filtration step.

12. The method of any preceding claim, wherein the cellular product comprises a biological compound or structure that is not released by the cells in the cell culture or wherein the cellular product comprises the cells themselves or a part thereof, optionally wherein the method comprises harvesting the cellular product by separating the cells from the hydrogel matrix and/or wherein harvesting the cellular product further comprises washing the cells cellularised structure, preferably wherein separating the cells from the hydrogel matrix is performed by mechanical separation and/or dissolution of the hydrogel matrix, and/or wherein harvesting the cellular product does not comprise a trypsination step.

13. The method of any preceding claim, wherein the hydrogel matrix is dissolvable using one or more enzymatic solution(s), optionally wherein the enzymatic solution(s) comprise one or more of collagenase (e.g. collagenase A), alginate lyase and dispase, and/or wherein the enzymatic solution(s) do(es) not include trypsin, and/or wherein the method comprises a step of dissolving the hydrogel matrix using one or more enzymatic solutions.

14. The method of any preceding claim, wherein the cell population and hydrogel matrix structure together form a cellularised structure and culturing the cell population at least partially embedded in a hydrogel matrix forming a three-dimensional structure comprises maintaining the cellularised structure in a liquid culture medium comprising at least a proportion of liquid medium in which the cell population has been culture prior to formation of the cellularised structure; and/or wherein the method further comprises providing a cellularised structure comprising the cell population and hydrogel matrix by consolidating a composition comprising the cell population and one or more biomaterials capable of forming a hydrogel, by cross-linking one or more of the biomaterials capable of forming a hydrogel, optionally wherein the cross-linking conditions are set to result in a degree of cross-linking that is compatible with the growth of the cells within the cross-linked matrix.

15. A system for performing a bioproduction process comprising the production of a cellular product by a cell population, the system comprising:

> a bioreactor and
> one or more cellularised structure(s) comprising a cell population at least partially embedded in a hydrogel matrix forming a three-dimensional structure,
> and optionally further comprising:

>> one of more flow devices to create a flow of medium contained in the bioreactor and in which the cellularised structure is immersed;
>> one or more support structures within the bioreactor to support the one or more cellularised structures;
>> one or more monitoring devices such as e.g. a camera, microscope, gas sensor, flow sensor, metabolite sensor, pH sensor, temperature sensor;
>> one or more supply and/or collection systems for supplying culture medium to the bioreactor and/or collecting culture medium from the bioreactor; and/or
>> a 3D printing system for depositing a bioink composition from which the cellularised structure is obtained.

Fig. 1A

120

PROVIDE BIO-INK COMPOSITION

130

DEPOSIT BIO-INK COMPOSITION BY ADDITIVE MANUFACTURING

CONSOLIDATE BIO-INK COMPOSITION — 140

150

CULTURE CELL POPULATION IN 3D HYDROGEL STRUCTURE

— 160

HARVEST CELLULAR PRODUCT

REMOVE / REPLACE CULTURE MEDIUM — 160A

SEPARATE CELLS FROM HYDROGEL MATRIX

160B

Fig. 1B

Fig. 2

200 CELLS AMPLIFICATION

*Suspension / adherent*
*Different cell types*

210 CELLS TRYPSINATION / HARVEST

220 BIOINK FORMULATION

Cells + Biomaterials
Gelatin
Alginate
Fibrinogen

230 3D BIOPRINTING

Pneumatic extrusion

240 HYDROGEL RETICULATION

1. Print
gelatin   Fibrinogen
Alginate

2. Consolidate

250 CELLULARIZED STRUCTURE CULTIVATION

260 DISSOCIATION PROCESS = CELL HARVESTING

EP 4 141 095 A1

30

Fig. 3

Fig. 4

Fig. 5

Fig. 6

Fig. 7

Fig. 8

Fig. 9

A

500µm      100µm

B

500µm      50µm

C

500µm      100µm

D

D16          D26          J49 & J5 post-infection with BacMam GFP

Fig. 10

Fig. 11

Fig. 12

Fig. 13

Full geometry
volume : 10.8 cm³

Full geometry
volume : 0.2 cm³

Full geometry
volume : 0.2 cm³

Full geometry
volume : 0.4 cm³

Porosity 0%          50%          0%                    50%          50%

Pores size :
0.4 x 0.4 mm

Pores size :
0.4 x 0.4 mm

Pores size :
1.2 x 1.2 mm

Fig. 14

Fig. 15A

Fig. 15B

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/283736 A1 (KANG HYUN WOOK [KR] ET AL) 10 September 2020 (2020-09-10) * paragraphs [0008], [0009], [0014], [0053] – [0072]; figures 1,2 * | 1–15 | INV. C12M1/12 C12M1/26 B33Y70/10 |
| X | US 2019/376016 A1 (LEI YUGUO [US]) 12 December 2019 (2019-12-12) * paragraphs [0013], [0014], [0018], [0020], [0021], [0027], [0028], [0030] – [0032], [0038], [0040] – [0043]; figures 1,2,4,5 * | 1–15 | |
| X | US 2018/230423 A1 (O'MAHONY AIDAN PATRICK [AU] ET AL) 16 August 2018 (2018-08-16) * paragraphs [0004], [0067] – [0069], [0084], [0100], [0102], [0132] – [0135], [0139], [0162], [0170], [0188], [0189]; figures 1-3 * | 1–15 | |

TECHNICAL FIELDS
SEARCHED      (IPC)

C12M
B33Y

The present search report has been drawn up for all claims

| Place of search | Date of completion of the search | Examiner |
|---|---|---|
| **Berlin** | **10 February 2022** | **Pantelidis, D** |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 6143

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2022

| Patent document cited in search report | | Publication date | Patent family member(s) | | Publication date |
|---|---|---|---|---|---|
| US 2020283736 | A1 | 10-09-2020 | EP | 3705294 A1 | 09-09-2020 |
| | | | KR | 102156310 B1 | 15-09-2020 |
| | | | US | 2020283736 A1 | 10-09-2020 |
| US 2019376016 | A1 | 12-12-2019 | AU | 2017363878 A1 | 13-06-2019 |
| | | | CA | 3044605 A1 | 31-05-2018 |
| | | | CN | 110213964 A | 06-09-2019 |
| | | | EP | 3544421 A1 | 02-10-2019 |
| | | | JP | 2019535284 A | 12-12-2019 |
| | | | US | 2019376016 A1 | 12-12-2019 |
| | | | WO | 2018098295 A1 | 31-05-2018 |
| US 2018230423 | A1 | 16-08-2018 | AU | 2016297675 A1 | 08-02-2018 |
| | | | CA | 2992811 A1 | 26-01-2017 |
| | | | CN | 107922921 A | 17-04-2018 |
| | | | EP | 3325611 A1 | 30-05-2018 |
| | | | JP | 6791960 B2 | 25-11-2020 |
| | | | JP | 2018522587 A | 16-08-2018 |
| | | | KR | 20180032597 A | 30-03-2018 |
| | | | US | 2018230423 A1 | 16-08-2018 |
| | | | WO | 2017011854 A1 | 26-01-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20190002836 A

- US 2019002836 A1

**Non-patent literature cited in the description**

- **POURCHET et al.** *Adv. Healthcare Mater.,* 2017, 1601101
- **POURCHET et al.** *Bioprinting,* December 2020, vol. 20, e00114

- **SAKHR et al.** *Int J Mol Sci,* April 2021, vol. 22 (7), 3290